(19) Europäisches Patentamt
European Patent Office
Office européen des brevets

(11) EP 2 099 915 B1

(12) **EUROPEAN PATENT SPECIFICATION**

(45) Date of publication and mention of the grant of the patent:
**14.09.2011 Bulletin 2011/37**

(21) Application number: **07857186.6**

(22) Date of filing: **28.12.2007**

(51) Int Cl.:
*C12N 15/82* (2006.01) *C08B 30/00* (2006.01)
*A21D 2/18* (2006.01) *A23L 1/0522* (2006.01)

(86) International application number:
**PCT/EP2007/011497**

(87) International publication number:
**WO 2008/080630 (10.07.2008 Gazette 2008/28)**

(54) **CORN STARCH AND ALSO CORN FLOURS AND FOOD COMPRISING THIS CORN STARCH**

MAISSTÄRKE SOWIE DIESE MAISSTÄRKE ENTHALTENDE MAISMEHLE UND LEBENSMITTEL

AMIDON DE MAÏS, ET FARINES DE MAÏS ET PRODUITS ALIMENTAIRES COMPRENANT LEDIT AMIDON DE MAÏS

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **29.12.2006 EP 06090228**
**09.01.2007 US 879377 P**
**06.11.2007 EP 07075964**
**08.11.2007 US 2390**

(43) Date of publication of application:
**16.09.2009 Bulletin 2009/38**

(73) Proprietor: **Bayer CropScience AG**
**40789 Monheim am Rhein (DE)**

(72) Inventor: **FROHBERG, Claus**
**14532 Kleinmachnow (DE)**

(74) Representative: **Beyer, Andreas et al**
**Bressel und Partner - Patentanwälte**
**Park Kolonnaden**
**Potsdamer Platz 10**
**10785 Berlin (DE)**

(56) References cited:
**EP-A- 1 362 919** **WO-A-97/45545**
**WO-A-99/66050**

- **APARICIO-SAGUILAN ALEJANDRO ET AL: "Thermal and viscoelastic properties of starch gels from maize varieties" JOURNAL OF THE SCIENCE OF FOOD AND AGRICULTURE, vol. 86, no. 7, May 2006 (2006-05), pages 1078-1086, XP002439315 ISSN: 0022-5142**
- **SIEVERT D ET AL: "THERMAL BEHAVIOR OF POTATO AMYLOSE AND ENZYME-RESISTANT STARCH FROM MAIZE" CEREAL CHEMISTRY, AACC INTERNATIONAL, ST PAUL, MN, US, vol. 70, no. 3, 1993, pages 333-338, XP009016197 ISSN: 0009-0352**
- **KOSSMANN J ET AL: "UNDERSTANDING AND INFLUENCING STARCH BIOCHEMISTRY" CRITICAL REVIEWS IN PLANT SCIENCES, CRC PRESS, BOCA RATON, FL, US, vol. 19, no. 3, May 2000 (2000-05), pages 171-226, XP009011204 ISSN: 0735-2689**
- **POLASKE NATHAN W ET AL: "Amylose determination of native high-amylose corn starches by differential scanning calorimetry" STARCH, vol. 57, no. 3-4, April 2005 (2005-04), pages 118-123, XP002439316 ISSN: 0038-9056**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 099 915 B1

Printed by Jouve, 75001 PARIS (FR)

## Description

The present invention relates to corn starches having an amylose content between 15% by weight and 40% by weight and a content of rapidly digestible starch between 5% by weight and 25% by weight, a content of resistant starch between 15 % by weight and 60 % by weight and a content of slowly digestible starch of between 20 % by weight and 60 % by weight, and also corn flours and foods comprising these corn starches or corn flours. In addition, the present invention relates to processes for producing said corn starches/corn flours and use thereof as resistant starch, as prebiotic or for producing foods having a reduced glycemic index.

The polysaccharide starch is made up of chemically uniform basic building blocks, the glucose molecules, but is a complex mixture of differing molecular forms which possess differences with respect to the degree of polymerization and branching, and therefore differ greatly from one another in their physicochemical properties. A differentiation is made between amylose starch, and essentially unbranched polymer of alpha-1,4-glycosidically linked glucose units, and amylopectin starch, a branched polymer in which the branches come about by the occurrence of additionally alpha-1,6-glycosidic linkages. A further essential difference between amylose and amylopectin is in the molecular weight. Whereas amylose, depending on origin of the starch, possesses a molecular weight of 5 x 105-106 Da, that of amylopectin is between 107 and 108 Da. The two macromolecules can be differentiated by their molecular weight and their different physicochemical properties, which can be made visible most simply by their different iodine binding properties.

Amylose has long been considered a linear polymer consisting of alpha-1,4-glycosidically linked alpha-D-glucose monomers. In more recent studies, however, the presence of a few alpha-1,6-gliycosidic branch points has been demonstrated (approximately 0.1 %) (Hizukuri and Takagi, Carbohydr. Res. 134, (1984), 1-10; Takeda et al., Carbohydr. Res. 132, (1984), 83-92).

Different methods of determining the amylose content are available which, for one and the same starch, can lead to different numerical values/measurements of amylose content. Some of these methods are based on the iodine-binding capacity of amylose which can be determined potentiometrically (Banks & Greenwood, in W. Banks & C.T. Greenwood, Starch and its components (pp. 51-66), Edinburgh, Edinburgh University Press), amperometrically (Larson et al., Analytical Chemistry 25(5), (1953), 802-804) or spectrophotometrically (Morrison & Laignelet, J. Cereal Sc. 1, (1983), 9-20).

The amylose content can also be determined calorimetrically by means of Differential Scanning Calorimetry (DSC) measurements (Kugimiya & Donovan, Journal of Food Science 46, (1981), 765-770; Sievert & Holm, Starch/Stärke 45(4), (1993), 136-139). In addition, there is the possibility of determining the amylose content via the use of size exclusion chromatography (SEC) of native or debranched starch. This method was recommended, in particular, for determining the amylose content of genetically modified starches (Gérard et al., Carbohydrate Polymers 44, (2001). 19-27).

The use of resistant starch (RS) is increasingly growing in importance in the food industry. Starch is chiefly digested in the small intestine by the enzyme alpha-amylase, which hydrolyses the alpha-1,4-glucosidic bonds of starch to form sugars. In contrast thereto, resistant starch is not digested in the small intestine by alpha-amylases, but passes into the large intestine, where it behaves similarly to dietary fiber. The body obtains energy only to a small extent from the breakdown of RS-comprising products. This energy supply solely relates to the oxidative breakdown of resorbed short-chain fatty acids from the large intestine. These short-chain fatty acids are end products of the carbohydrate metabolism of the intestinal microflora. Substrates for the energy metabolism of the intestinal microflora and the large intestine epithelial cells is provided with the intake of RS-comprising foods. Large intestinal epithelial cells, to maintain their structure and function, have to resort to the luminal supply of short-chain fatty acids and, in particular, butyrate. Resistant starch is apparently a factor for prevention of diverticulosis and large bowel cancer.

A distinction is made between the following types of resistant starch:

- RS1 starch physically inaccessible to digestion, for example starch embedded in a protein or fiber matrix. If this is disintegrated physically (for example by chewing) or chemically (for example by breaking down the matrix surrounding it), it can be processed in a usual manner by the digestive juices.
- RS2 indigestible intact (granular) native starch grains, for example uncooked potato starch or banana starch, in particular from unripe bananas)
- RS3 indigestible retrograded starch which is not granular
- RS4 indigestible chemically modified starch, for example by crosslinking or esterification (acetylation etc.)

In contrast to RS4, the RS forms 1 to 3 can be made accessible to alpha-amylase breakdown by dissolution in NaOH or dimethyl sulfoxide.

For production of resistant starch, various processes have been described. Most of these processes relate to the production of RS3 starches (EP 564893 A1; EP 688872 A1; EP 846704 A1; US 5051271). All of these processes for producing resistant starch comprise the dispersion and gelatinization of starch in large excesses of water, followed by retrogradation with the use of enzymes or acids. They are based on the opinion that resistant starch is formed when the amylose fraction of starch is retrograded after the gelatinization of starch. It is assumed that the linear amylose molecules, after gelatinization, arrange themselves to form tight double-helix configurations bound by hydrogen bonds, so that the alpha-1,4-glucoside bonds are no longer accessible to alpha-amylases. These processes are labor-intensive, time-consuming, and can lead to low yields. Furthermore, the high water content of the products can make expensive drying steps necessary.

Granular starches of the RS2 type having a high content of resistant starch are found, especially, in native, uncooked wild type potato starches which, according to the method of determination, have an RS content of 74-85% by weight (Faisant et al., Sciences des Aliments 15, (1995), 83-89; Evans and Thompson, Cereal Chemistry 81(1), (2004), 31-37). Previously known granular corn starches having a high RS fraction are always distinguished by a high amylose content (>40% by weight): For native corn starches, i.e. granular corn starches, having a high amylose content, which are synthesized in various corn plants of the genotype amylose extender ("ae"), using the method of determining RS of Englyst et al. (Europ. J. of Clinical Nutrition 46 (Suppl. 2), (1992), pages 33-50), RS values of about 40-70% by weight were determined (Evans and Thompson, Cereal Chemistry 81(1), (2004), 31-37). The RS contents determined by Faisant et al. using two other methods of determining RS, for native, i.e. granular, amylomaize starch of the type Hylon VII (identical to ae VII which was investigated by Evans and Thompson) are, at approximately 54% by weight and 67% by weight, in this range which is also confirmed by an interlaboratory study which, using different methods of determination of RS, gives RS values for native amylomaize starch between about 50 and 72% by weight (McCleary and Monaghan, J. AOAC Int. 85, (2002), 665-675). Such granular amylomaize starches from amylose extender (ae) mutants have, for certain product groups, the disadvantage of poor processing properties, because these starches scarcely gelatinize, and have a low solubility and low swelling capacity. For applications in which only gelatinized starches are usable, or which require soluble starches or starches having swelling capacity, the amylomaize starches are therefore either not suitable at all or they must be additionally chemically modified in order to meet these requirements, which is time-consuming and costly (Senti and Russell, Tappi Vol. 43, No. 4, (April 1960), 343-349; Z. Luo et al., Starch/Stärke 58, (2006), 468-474).

In contrast thereto, conventional granular corn starches which originate from wild type corn plants which do not have the amylose extender genotype, are distinguished by an amylose content of approximately 24-29% by weight and an RS content which is about 24% by weight (determined according to the method of Englyst et al., see above).

Corn plants having the waxy genotype (also designated "wx") synthesize a granular corn starch which essentially consists of amylopectin. The RS content of this waxy corn starch is about 5% by weight (Evans and Thompson, Cereal Chemistry 81(1), (2004), 31-37).

Owing to the low fraction of resistant starch, therefore neither the granular corn starches from wild type corn plants nor those from waxy corn plants are suitable for use as (prebiotic) additive in the food industry. Other corn mutants which, compared with wild type corn plants, synthesize a starch having an increased fraction of resistant starch, are not currently known.

For determination of the RS content, use is made of different methods (for example Englyst et al., Eur. J. Clin. Nutr. 46 (Supplement), (1992), S33-S50, Faisant et al., Sci. Aliment. 15, (1995), 83-89; Champ et al., in Advanced Dietary Fibre Technology, B.V. McCleary & Prosky (Eds), Blackwell Science Ltd, Oxford, UK, pp. 106-119; Goni et al., Food Chem. 56, (1996), 445-449; Berry, J. Cereal Sci. 4, (1986), 301-314; McCleary and Monaghan, J. AOAC Int. 85, (2002), 665-675; McCleary et al., J. AOAC Int. 85, (2002), 1103-1111; Approved Methods of the American Association of Cereal Chemists, Tenth Edition, Volume I, (2000), ISBN 1-891127-12-8, AACC Method 32-40) which can lead to differing RS values for a given sample (for example Baghurst et al., Supplement to Food Australia 48(3), (1996), S3-S35). This is especially due to the fact that the four different RS types RS1 to RS4 differ very greatly from one another in their physicochemical properties, so that they cannot be determined by a single generally valid method (Baghurst et al., Supplement to Food Australia 48(3), (1996), S3-S35). For example, the AOAC/AACC method for determining the content of "dietary fibers" (=DF) provides a grinding step which leads to destruction of RS1 structures. Some methods are unsuitable for determining the content of RS2 starch, because they provide a step at high temperatures (100˚C) with the use of heat-stable alpha-amylases which inescapably leads to gelatinization of the starches which therefore lose their granular structure and therefore their RS2 structure (Delcour and Eerlingen, Cereal Foods World 41(2), (1996), 85-86).

In addition to resistant starches (RS), starches having a high fraction of slowly digestible starch (SDS) and/or starches having a low fraction of rapidly digestible starch (RDS), are also increasingly in demand in food preparation. This is because there is the suspicion that continuing consumption of foods having a high glycemic loading such as, for example, in the case of conventional starchy foods having a relatively high RDS fraction, and the resultant insulin secretion is a risk factor in the occurrence of diseases such as hypertension, overweight, heart disorders and diabetes type II. Foods having a high RDS fraction generally have a high glycemic index (GI) (Englyst et al., British Journal of Nutrition, 75, 327-337).

The rapid release of relatively large amounts of glucose which may be observed in the digestion of conventional starches, and absorption of said glucose via the small bowel epithelium leads to an abrupt increase in the blood sugar level and to secretion of insulin (insulin response). If the SDS content of a starch is increased and/or the RDS content of a starch is decreased, this leads to a prolonged release of glucose from the starch, to an altered insulin response and therefore finally to a reduction of the risk of the abovementioned disorders.

The use of starches having a high fraction of SDS and/or low fraction of RDS appears desirable in those foods in which continuous release of glucose is sought, such as, for example, in the case of athletes foods for endurance sport or in the case of dietetic food for reducing the feeling of hunger.

It is an object of the present invention to provide (granular) corn starches or corn flours which comprise this (granular) corn starch which, compared with the known (granular) corn starches or corn flours, have altered digestion properties (e.g. altered proportion of RS, SDS and/or RDS) and, if appropriate, altered thermal properties and, if appropriate, altered processing properties.

This object is achieved by the embodiments described in the claims.

The present invention relates to a corn starch which has

- an apparent amylose content between 15% by weight and 40% by weight, determined according to the method of Juliano, 1971, Cereal Science Today 16,10, p. 334-340, and

- a content of resistant starch (RS), determined by the method of Englyst et al., Europ. J. of Clinical Nutrition 46, Suppl. 2, 1992, p. 33-50, of between 15% by weight and 60% by weight, and

- a content of rapidly digestible starch (RDS) based on the amount of starch in dry weight of between 5% by weight and 25% by weight, wherein the content of RDS is the fraction of a corn starch which is released as glucose after 20 minutes in the method of Englyst et al. for determining the RS content and,

- a content of slowly digestible starch (SDS) based on the amount of starch in dry weight of between 20% by weight and 60% by weight, wherein the content of SDS is the fraction of a corn starch which is released as glucose after 2 hours in the method of Englyst et al. minus the glucose released after 20 minutes,

wherein the starch is obtained by extraction from a corn plant which expresses a heterologous starch synthase II from the genus triticum.

The present invention also relates to a corn starch, preferably a granular corn starch which has an amylose content preferably between 18% by weight and 35% by weight, and particularly preferably between 20% by weight and 30% by weight, and a content of rapidly digestible starch (RDS) preferably between 7% by weight and 23% by weight, more preferably between 10% by weight and 23% by weight, particularly preferably between 10% by weight and 17% by weight.

In a further embodiment, the corn starch, preferably granular corn starch of the invention has a content of rapidly digestible starch (RDS) between 10% by weight and 23% by weight, preferably between 12% by weight and 21 % by weight, particularly preferably between 14% by weight and 20% by weight.

In a preferred embodiment, the corn starch of the invention, preferably granular corn starch, has a content of rapidly digestible starch (RDS) between 7% by weight and 25% by weight, preferably between 10% by weight and 23% by weight, particularly preferably between 10% by weight and 17% by weight.

In a further aspect, the corn starch of the application, preferably granular corn starch, has a content of slowly digestible starch (SDS) between 22% by weight and 67% by weight, preferably between 27% by weight and 63% by weight, particularly preferably between 35% by weight and 50% by weight.

The present application also relates to a corn starch, preferably a granular corn starch, which has an amylose content between 15% by weight and 40% by weight, preferably between 18% by weight and 35% by weight, and particularly preferably between 20% by weight and 30% by weight, and a content of slowly digestible starch (SDS) between 22% by weight and 67% by weight, preferably between 27% by weight and 63% by weight, particularly preferably between 35% by weight and 50% by weight.

In a preferred embodiment, the corn starch of the invention, preferably granular corn starch, has a content of slowly digestible starch (SDS.) between 20% by weight and 60% by weight, preferably between 30% by weight and 56% by weight, particularly preferably between 40% by weight and 50% by weight.

Surprisingly, the content of slowly digestible starch (SDS) in the corn starches of the invention, preferably granular corn starches, is increased by at least 200%, preferably by 220%-400%, particularly preferably by 240%-300%, compared with corresponding corn starches from amylose-extender (ae) corn plants (such as, for example, Hylon®7) and/or reduced by at least 5% compared with corresponding corn starches from wild type corn plants, preferably by 7%-40%, particularly preferably by 10%-30%.

In a further embodiment, the corn starch of the invention, which is preferably a granular corn starch, has an RS content between 26% by weight and 55% by weight, preferably between 27% by weight and 50% by weight, particularly preferably between 40% by weight and 48% by weight.

In a further embodiment, the corn starch of the invention, which is preferably a granular corn starch, has an RS content between 26% by weight and 45% by weight, preferably between 27% by weight and 40% by weight, particularly preferably between 30% by weight and 36% by weight.

In a further embodiment, the corn starch of the invention, which is preferably a granular corn starch, has an RS content between 15% by weight and 60% by weight, preferably between 29% by weight and 50% by weight, particularly preferably between 35% by weight and 48% by weight.

In the context of the present invention, the RS content is determined by the method of Englyst et al. (Europ. J. Clinical Nutrition 46 (Suppl. 2), (1992), S33-50, see, in particular, the following sections from Englyst et al., page S35-S36: "Reagents Apparatus, Spectrophotometer"; page S36-S37, paragraph "Measurement of free glucose (FG)"; page S38, paragraph "Measurement of RDS and SDS"), most preferably the RS content is determined by the laboratory scale method described below in the method section "13) Determination of the resistant starch fraction (digestibility)".

The resistant starch (RS) fraction of the starch is described as the fraction of the weighed starch sample (dry weight) which is not released as glucose in the method described after 2 hours. It is accordingly given by the following formula:

$$\text{RS starch in \%} = 100\% - 100\% \times (\text{glucose released after 2 h in mg/dry weight of starch in mg})$$

In the context of the present invention, "the rapidly digestible starch (RDS) content" is to be taken to mean the fraction of a corn starch which is released as glucose after 20 minutes in the method cited above of Englyst et al. for determining the RS content. In the context of the present invention the RDS content is preferably determined by the laboratory scale method, which is described below in the method section "13) Determination of the resistant starch fraction (digestibility)".

The report in percent by weight in this case relates to dry weight of the starch sample. Accordingly, in the context of the present invention, the following applies:

$$\text{RDS in \%} = 100\% \times (\text{glucose released after 20 minutes in mg/dry weight of starch in mg})$$

In the context of the present invention, "the content of slowly digestible starch (SDS)" is taken to mean the fraction of a corn starch which is released as glucose after 2 hours in the abovementioned method of Englyst et al., minus the glucose released after 20 minutes (RDS). In the context of the present invention the SDS content is preferably determined by the laboratory scale method, which is described herein below in the method section "13) Determination of the resistant starch fraction (digestibility)".

The figure in percent by weight in this case relates to the dry weight of the starch sample. Accordingly, in the context of the present invention:

$$\text{SDS in \%} = 100\% \times (\text{glucose released after 2 h in mg/dry weight of starch in mg}) \text{ minus } (\text{glucose released after 20min in mg/dry weight of starch in mg})$$

In the context of the present invention, the sum of the content of SDS, the content of RDS and the content of RS is 100%.

A "granular corn starch", in the context of the present invention, is to be taken to mean a corn starch which has not been gelatinized, or not completely gelatinized, and predominantly has a granular structure, i.e. a least 90%, preferably at least 95%, particularly preferably at least 99% of the starch grains of a starch sample have a granular shape. Completely retrograded corn starch is not a granular corn starch within the meaning of the present invention. The granular structure of a corn starch grain leads, in the light microscope, under polarized light, to a characteristic light birefringence and can be determined hereby (see, for example, page 126, figure 4 In Yahl at al., Microscope 32, (1984), 123-132).

In the context of the present Invention, the amylose content Is taken to mean the content of apparent amylose. The amylose content is determined by the method described herein below "determination of the apparent amylose content" (Juliano, 1971, Cereal Science Today 16(10), 334-340).

The thermal properties of the corn starch of the invention and also of the corn flour of the invention may be analyzed by differential scanning calorimetry (DSC). The thermal properties are represented as gelatinization temperature with the values for the DSC T-onset (= lowest temperature of gelatinization) and for DSC T-peak (= maximum gelatinization temperature).

The expression "DSC T-onset temperature", in the context of the present invention, is to be taken to mean that temperature which represents the start of phase conversion of the starch or flour sample. It is characterized as the intersection between the extension of the baseline and the tangent to the rising flank of the peak through the transition point.

The expression "DSC T-peak temperature", in the context of the present invention, denotes the temperature at which the DSC curve of the starch sample or flour sample has reached a maximum and the first derivative of the curve is zero.

The "DSC T-onset" temperature and also the "DSC T-peak" temperature are determined, in the context of the present invention, by the method described below ("thermal analysis of corn flour/starch by means of differential scanning calorimetry (DSC)").

The present invention, in a further embodiment, discloses a corn starch, which is preferably a granular corn starch, that has an amylose content between 15% by weight and 40% by weight, preferably between 18% by weight and 35% by weight, and particularly preferably between 20% by weight and 30% by weight, and has a DSC T-onset temperature between 70.5˚C and 77.5˚C, preferably between 71.0˚C and 76.5˚C, particularly preferably between 71.5˚C and 75.5˚C.

The present invention, in a further embodiment, discloses a corn starch, which is preferably a granular corn starch, that has an amylose content between 15% by weight and 40% by weight, preferably between 18% by weight and 35% by weight, and particularly preferably between 20% by weight and 30% by weight, and has a DSC T-onset temperature between 67.5˚C and 75.0˚C, preferably between 68.0˚C and 74.5˚C, particularly preferably between 68.5˚C and 74.0˚C.

The present Invention, In a further embodiment, discloses a corn starch, which is preferably a granular corn starch, that has an amylose content between 15% by weight and 40% by weight, preferably between 18% by weight and 35% by weight, and particularly preferably between 20% by weight and 30% by weight, and has a DSC T-peak temperature between 74.0˚C and 82.0˚C, preferably between 75.0˚C and 79.5˚C, particularly preferably between 77.0˚C and 79.0˚C.

In a further embodiment, the corn starch disclosed in the invention, which is preferably a granular corn starch, has a DSC T-peak temperature between 75.5˚C and 84.5˚C, preferably between 76.5˚C and 81.5˚C, particularly preferably between 77.5˚C and 80.5˚C.

In a further embodiment, the present invention discloses a corn starch, which is preferably a granular corn starch which, in addition to an amylose content between 15% by weight and 40% by weight, preferably between 18% by weight and 35% by weight, and particularly preferably between 20% by weight and 30% by weight, in addition, optionally has

a) an RS content between 26% by weight and 45% by weight, preferably between 27% by weight and 40% by weight, particularly preferably between 30% and 36% by weight and/or
b) a rapidly digestible starch (RDS) content based on the amount of starch (dry weight) between 10% by weight and 23% by weight, preferably between 12% by weight and 21% by weight, particularly preferably between 14% by weight and 20% by weight and/or
c) a DSC T-onset temperature between 70.5˚C and 77.5˚C, preferably between 71.0˚C and 76.5˚C, particularly preferably between 71.5˚C and 75.5˚C and/or
d) a DSC T-peak temperature between 75.5˚C and 84.5˚C, preferably between 76.5˚C and 81,5˚C, particularly preferably between 77.5˚C and 80.5˚C.

In a further aspect, the present application discloses a corn starch, preferably a granular corn starch, which, in addition to an amylose content between 15% by weight and 40% by weight, preferably between 18% by weight and 35% by weight, and particularly preferably between 20% by weight and 30% by weight, optionally additionally has

a) an RS content between 26% by weight and 55% by weight, preferably between 27% by weight and 50% by weight, particularly preferably between 40% by weight and 48% by weight, and/or
b) a rapidly digestible starch (RDS) content based on the amount of starch (dry weight), between 5% by weight and 25% by weight, between 7% by weight and 23% by weight, preferably between 10% by weight and 23% by weight, particularly preferably between 10% by weight and 17% by weight, and/or

c) a slowly digestible starch (SDS) content based on the amount of starch (dry weight) between 22% by weight and 67% by weight, preferably between 27% by weight and 63% by weight, particularly preferably between 35% by weight and 50% by weight, and/or

d) a DSC T-onset temperature between 67.5˚C and 75.0˚C, preferably between 68.0˚C and 74.5˚C, particularly preferably between 68.5˚C and 74.0˚C, and/or

e) a DSC T-peak temperature between 75.5˚C and 84.5˚C, preferably between 76.5˚C and 81.5˚C, particularly preferably between 77.5˚C and 80.5˚C.

In a further embodiment, the present invention discloses a corn starch, preferably a granular corn starch, which, in addition to an amylose content between 15% by weight and 40% by weight, preferably between 18% by weight and 35% by weight, and particularly preferably between 20% by weight and 30% by weight, optionally additionally has

a) an RS content between 15% by weight and 60% by weight, preferably between 29% by weight and 50% by weight, particularly preferably between 35% by weight and 48% by weight, and/or

b) a rapidly digestible starch (RDS) content based on the amount of starch (dry weight) between 5% by weight and 25% by weight, between 7% by weight and 25% by weight, preferably between 10% by weight and 23% by weight, particularly preferably between 10% by weight and 17% by weight, and/or

c) a slowly digestible starch (SDS) content based on the amount of starch (dry weight) between 20% by weight and 60% by weight, preferably between 30% by weight and 56% by weight, particularly preferably between 40% by weight and 50% by weight, and/or

d) a DSC T-onset temperature between 67.5˚0 and 75.0˚C, preferably between 68.0˚C and 74.5˚C, particularly preferably between 68.5˚C and 74.0˚C, and/or

e) a DSC T-peak temperature between 75.5˚C and 84.5˚C, preferably between 76.5˚C and 81.5˚C, particularly preferably between 77.5˚C and 80.5˚C.

In a further embodiment, the corn starch disclosed in the invention exhibits a side chain distribution of the amylopectin side chains which is altered compared with the side chain distribution of wild type corn starch.

In a further embodiment, the corn starch disclosed in the invention shows an increase in the content of the amylopectin side chains having a degree of polymerization (dp) of dp 17-20 by 2%-20%, and preferably by 5%-15%, particulaly preferably by 6%-10%, compared with the content of the corresponding side chains of the amylopectin of corresponding wild type corn plants.

In a further embodiment, the corn starch disclosed in the invention shows a decrease in the content of amylopectin side chains having a degree of polymerization (dp) of dp 6-11 by 5%-60% , and preferably by 15%-45%, compared with the content of the corresponding amylopectin side chains of corresponding wild type corn plants.

In a further embodiment, the corn starch disclosed in the invention shows a reduction of the content of amylopectin side chains having a degree of polymerization (dp) of dp 6-11 by 3%-50%, preferably by 5%-30%, and particularly preferably by 6%-20%, compared with the content of the corresponding amylopectin side chains of corresponding wild type corn plants.

The side chain distribution, in the context of the present invention, is determined according to the method described below ("preparation of corn flour/com starch for studying the amylopectin side chain distribution by means of high-pressure anion-exchange chromatography"). The fraction of side chains is determined via determining the percentage fraction of a certain side chain of the total fraction of all side chains. The total fraction of all side chains is determined via determination of the total area below the peaks which represent degrees of polymerization of DP 6 to 32 in the HPLC chromatogram. The percentage fraction of a defined side chain of the total fraction of all side chains is determined by determining the ratio of the area under the peak which represents this side chain in the HPLC chromatogram to the total area. For determination of peak areas, for example, use can be made of the program Chromelion 6.60 from Dionex, USA.

In a further embodiment, the present invention discloses the use of the corn starch of the invention, which is preferably a granular corn starch, as resistant starch.

In a further embodiment, the present invention relates to the use of the corn starch of the invention, which is preferably a granular corn starch, as prebiotic. This is because the corn starch of the invention surprisingly displays an increased RS content compared with corn starch of wild type corn plants which had previously been found only for corn starches having an amylose content of greater than 40% by weight and there led to a prebiotic effect (for example van Munster et al., Digestive Diseases and Sciences 39(4), (1994), 834-842).

The corn starches/corn flours of the invention, in addition have the advantage of a reduced fraction of rapidly digestible flour or starch (RDS), which is particularly advantageous, since rapid release of relatively large amounts of glucose and absorption thereof via the small bowel epithelium leads to an abrupt increase in the blood sugar level. In consequence thereof, there is a secretion of insulin (insulin response). The continuing consumption of foods having a high glycemic loading and the resultant insulin secretion are suspected to be a risk factor in the occurrence of diseases such as hypertension, overweight, heart disorders and diabetes type II,

In a further embodiment, the present invention therefore relates to the use of the corn starch of the invention, which is preferably a granular corn starch, or of the corn flour of the invention described herein below for producing a food which has a reduced glycemic index compared with the glycemic index of a food which comprises starch or flour from corresponding wild type corn plants, preferably a food suitable for the Nutrition of diabetics or for the prevention of hypertension, overweight, heart disorders or diabetes type II. On account of the replacement of conventional corn starch or corn flour, for example from wild type corn plants, by the corn starch of the invention or the corn flour of the invention, the food exhibits a reduced glycemic index which is due to the fact that the corn starch of the invention/the corn flour of the invention, compared with starch/flour from wild type corn plants, has a significantly reduced content of rapidly digestible starch (RDS).

In a further embodiment, the present invention discloses the use of the, preferably granular, corn starch of the invention or of the corn flour of the invention as component of diabetic food or for prevention of hypertension, overweight, heart disorders or diabetes type II.

In a further embodiment, the present invention relates to the use of the, preferably granular, corn starch of the invention, or of the corn flour of the invention, for reducing the glycemic index of foods compared with the glycemic index of foods which comprise starch or flour from corresponding wild type corn plants.

The glycemic index (GI) is a measure of determining the effect of a carbohydrate-comprising food on the blood sugar level. The glycemic index gives in figures the effect of the carbohydrates or the food which increases the blood sugar. The effect of glucose or white bread on increasing blood sugar generally acts here as a reference value (100).

To determine the GI of a food, the blood sugar course is measured in test subjects after a meal, generally over a period of 2 hours. For this purpose the subjects receive the food whose GI is to be determined in an amount which comprises exactly 50 grams of utilizable carbohydrates. After the test "meal", the blood sugar Is measured regularly and its course is thereby observed. Measurements are taken of a plurality of test subjects and a mean is calculated in order to take into account blood sugar curves differing from person to person. The areas under the blood sugar curves are integrated. The area resulting after the intake of glucose (usually 50 grams) (= reference food), as the standard, is set at 100. The GI for a food therefore describes the relative area under the blood sugar curve compared with the curve after the reference food (glucose) as a percentage.

Detailed descriptions of methods of determining the glycemic index are known to those skilled in the art and are described, for example, by Wolever et al. (Am. J. Clin. Nutr. 54, (1991), 846-854) or in FAO Food and Nutrition Paper 66, "Carbohydrates in human nutrition", Chapter 4-The Role of the Glycemic Index in Food Choice, pp. 25-30, Report from Apr. 14-18, (1997).

A high GI means that the carbohydrates of the food are rapidly broken down to glucose and passed into the blood, so that the blood sugar level increases rapidly, and a high regulatory insulin secretion proceeds. Foods having a medium or low GI, in contrast, produce only a slower and overall lower rise in the blood sugar curve.

In a further embodiment, the present invention discloses the use of the, preferably granular, corn starch of the invention or of the corn flour of the invention for producing foods which, after intake by the human body, lead to a slower increase in the blood sugar level than is the case after intake of corresponding foods which comprise starch/flour from (corresponding) wild type corn plants.

Compared with the previously known corn starches, the corn starches of the invention in addition have the advantage that, in addition to a reduced fraction of RDS compared with wild type corn starches, they simultaneously have an increased fraction of SDS compared with corn starches of amylose extender mutants. The advantages accompanying this increase in the SDS fraction of the starch such as, for example, a retardation of glucose release (for example athletes food) and a prolonged feeling of saturation (for example weight management), are therefore coupled with the above described advantages which are due to the decreased RDS fraction of the starch (for example decreased glycemic response).

Typical foods to which the starch of the invention/the flour of the invention can be added comprise tortillas, tortilla chips, bakery products (for example bread, corn bread, rolls, biscuits, cakes, waffles, muffins, tacos), pancakes, pizza, polenta, enchiladas, pasta (for example noodles), "cornmeal mush" (USA), "porridge" (GB), stews, sauces, corn flour pudding, milk products (for example yoghurt, quark), puddings, spreads (for example butter, margarine), drinks, drink powders, prepared dishes, sauces, (breakfast) cereals and others.

In a further embodiment, the present invention relates to a process for producing a - preferably granular - corn starch of the invention which comprises the step of extracting the starch from a corn plant which expresses a heterologous starch synthase II.

In a further embodiment, the present invention further discloses a process for producing a starch which comprises the step of extracting the starch from a corn plant cell which expresses a heterologous starch synthase II. In a further embodiment of the present invention, the corn starch of the invention is extracted from a corn plant comprising such corn plant cells, from reproductive material of such a corn plant and/or from starch-storing parts of such a corn plant. Preferably, the process disclosed in the invention also comprises the step of harvesting the cultivated corn plants or of the starch-storing plant parts and/or the reproductive material of these corn plants before extraction of the starch. In a further embodiment, the process disclosed in the invention also comprises the step of cultivating the corn plants before harvesting.

Compared with conventional processes for producing corn starches having increased thermal stability and/or altered digestion properties using corn mutants (for example ae or ae wx mutants) whose starch yield, compared with wild type corn plants can be considerably decreased, the process has the advantage that the expression of the heterologous starch synthase II in corn has no such losses in starch yield as a consequence.

Processes for extracting starch from plants or from starch-storing parts of corn plants are known to those skilled in the art. In addition, processes are described for extracting starch from various starch-storing plants, for example in Starch: Chemistry and Technology (edited- Whistler, BeMiller and Paschall (1994), 2nd Edition, Academic Press Inc. London Ltd; ISBN 0-12-746270-8; see, for example, chapter XII, pages 412-468: corn and sorghum starches: production; by Watson; Eckhoff et al., Cereal Chem. 73 (1998), 54-57). Corn starch is generally extracted on an industrial scale by what is termed wet milling. Devices which are customarily used for extracting starch from plant material are separators, decanters, hydrocyclones, spray dryers and fluidized-bed dryers.

The expression "starch-storage parts", in the context of the present invention, is to be taken to mean those parts of a plant in which starch, in contrast to transitory leaf starch, is stored as a deposit for surviving relatively long periods of time. Preferred starch-storage plant parts are corn kernels, particular preference is given to corn kernels comprising an endosperm.

Proteins having the activity of a starch synthase II (ADP-glucose-1,4-alpha-D-glucan 4-alpha-D-glucosyltransferase; EC 2.4.1.21) have in their structure a sequence of defined domains. At the N terminus, they have a signal peptide for transport into plastids. In the direction of the N terminus towards the C terminus, there follow an N-terminal region and a catalytic (domain. (Li et al., 2003, Funct Integr Genomics 3, 76-85). Further analyses based on amino acid sequence comparisons (http://hits.isb-sib.ch/cgi-bin/PFSCAN) of various proteins having the activity of a starch synthase II found that these proteins have three specific domains. In the amino acid sequence represented under SEQ ID NO 2, the amino acids 322 to 351 are domain 1, the amino acids 423 to 462 are domain 2 and amino acids 641 to 705 are domain 3. Domain 1 is coded by the nucleotides 1190 to 1279, domain 2 by the nucleotides 1493 to 1612 and domain 3 by the nucleotides 2147 to 2350 of the nucleic acid sequence represented under SEQ ID NO 1.

In the context of the present invention, the expression "starch synthase II" is taken to mean a protein which catalyses a glucosylation reaction in which glucose radicals of the substrate ADP-glucose are transferred to alpha-1,4-linked glucan chains, with formation of an alpha-1,4-link (ADP-glucose + {(1,4)-alpha-D-glucosyl}(N) <=> ADP + {(1,4)-alpha-D-glucosyl}(N+1)). The amino acid sequence of starch synthase II exhibits an identity of at least 86%, preferably at least 93%, particularly preferably at least 95%, with amino acids 322 to 351 (domain 1) of the amino acid sequence represented under SEQ ID NO 2 and/or an identity of at least 83%, preferably at least 86%, particularly preferably at least 95%, with amino acids 423 to 462 (domain 2) of the amino acid sequence represented under SEQ ID NO 2 and/or an identity of at least 70%, preferably at least 82%, preferably 86%, particularly preferably 98%, in particular preferably at least 95%, with amino acids 641 to 705 (domain 3) of the amino acid sequence represented under SEQ ID NO 2.

The expression "identity", in the context of the present invention, is to be taken to mean the number of amino acids/nucleotides in agreement (identity) with other proteins/nucleic acids, expressed in percent. Preferably, the identity of a protein having the activity of a starch synthase II is determined by comparison with the amino acid sequence reported under SEQ ID NO 2, or the identity of a nucleic acid molecule encoding a protein having the activity of a starch synthase II is determined by comparison with the nucleic acid sequence reported under SEQ ID NO 1 with other proteins/nucleic acids by computer programs. If sequences which are compared with one another have different lengths, the identity must be determined in such a manner that the number of amino acids/nucleotides which the shorter sequence has in common with the longer sequence determines the percentage fraction of the identity. Preferably, the identity is determined by means of the known and publicly available computer program ClustalW (Thompson et al., Nucleic Acids Research 22 (1994), 4673-4680). ClustalW is publicly available from Julie Thompson (Thompson@EMBL-Heidelberg.DE) and Toby Gibson (Gibson@EMBL-Heidelberg.DE), European Molecular Biology Laboratory, Meyerhofstrasse 1, D 69117 Heidelberg, Germany. ClustalW can likewise be downloaded from various internet sites, inter alia from the IGBMC (Institut de Génétique et de Biologie Moléculaire et Cellulaire, B.P.163, 67404 IIlkirch Cedex, France; ftp://ftp-igbmc.u-strasbg.fr/pub/) and from the EBI (ftp://ftp.ebi.ac.uk/pub/software/) and also from all mirrored internet sites of the EBI (European Bioinformatics Institute, Wellcome Trust Genome Campus, Hinxton, Cambridge CB10 1 SD, UK).

Preferably, version 1.8 of the ClustalW computer program is used to determine the identity between proteins described in the context of the present invention and other proteins. In this procedure the following parameters must be set: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP. Preferably, version 1.8 of the ClustalW computer program is used in order to determine the identity between, for example, the nucleotide sequence of the nucleic acid molecules described in the context of the present invention and the nucleotide sequence of other nucleic acid molecules. In this process the following parameters must be set: KTUPLE=2, TOPDIAGS=4, PAIRGAP=5, DNAMATRIX:IUB, GAPOPEN=10, GAPEXT=5, MAXDIV=40, TRANSITIONS: unweighted.

"Heterologous" starch synthase II, in the context of the present invention, is to be taken to mean a starch synthase II which does not occur naturally in the corn plant (cell), but whose encoding DNA sequence is introduced into the cell, for example, by genetic engineering methods such as, for example, transformation of the cell. In this process the encoding DNA sequence of the heterologous starch synthase II can originate from another corn variety than the transformed corn plant cell and is in this case preferably not under the control of its own promoters. Preferably, the heterologous starch synthase is from a different plant species than the transformed corn plant cell or corn plant or the starch synthase II used is not under the control of its own promoter. Particularly preferably, the encoding DNA sequence of the heterologous starch synthase II originates from a different plant genus than the transformed corn plant cell or corn plant.

The expression "plant genus", in the context of the present invention, is to be taken to mean a hierarchical stage of biological systematics. A genus comprises one or more species. One example of a genus is *Triticum L.* (wheat). All species within a genus always have a two-part (binominal) name which, in addition to the genus name, also comprises a specific epithet. *Triticum aestivum* L. (soft wheat) is accordingly a species of the genus *Triticum.*

Nucleic acid sequences and the amino acid sequences corresponding thereto which exhibit the required identity with domains 1, 2 and 3 and which encode a starch synthase II are known to those skilled in the art and are published, for example, by Gao and Chibbar (Genome 43 (5), (2000), 768-775: starch synthase II from wheat NCBI Acc No. AJ269502.1, AJ269503.1, AJ269504.1) or under Accession No. AF155217.2 (*Triticum aestivum*), AY133249 (*Hordeum vulgare*), Accession No. AY133248 (*Aegilops tauschii*), Accession Nos XP467757, AAK64284 (*Oryza sativa*), Accession No. AAK81729 (*Oryza sativa*), Accession Nos AAD13341, AAS77569, Accession No. AAF13168 (*Manihut esculenta*), Accession No. AAP41030 (*Colocasia esculenta*), Accession No. AAS88880 (*Ostraeococcus taun*), or Accession No. AAC17970 (*Chlamydomonas reinhardii*). Said nucleic acid sequences and amino acid sequences encoding a protein having the activity of a starch synthase II are accessible via NCBI (http://www.ncbi.nlm.nih.gov/entrez/) and are hereby explicitly incorporated into the contents of the present application by naming the references.

In a particularly preferred embodiment, in the context of the present invention, use is made of a starch synthase II of the genus *Triticum,* preferably of the species *Triticum aestivum.* Particular preference is given to starch synthase II having the amino acid sequence reported under SEQ ID NO 2 or the nucleotide sequence reported under SEQ ID NO 1.

In a further embodiment of the present invention, the corn plant (cell) which synthesizes the corn starch of the invention is genetically modified, wherein the genetic modification leads to an increase in the activity of a starch synthase II compared with corresponding wild type corn plant cells or wild type corn plants which are not genetically modified.

The genetic modification in this case can be any genetic modification which leads to an increase in the activity of a starch synthase II compared with corresponding wild type corn plant cells or wild type corn plants which are not genetically modified.

The expression "wild type corn plant cell", in the context of the present invention, means that these are corn plant cells which served as starting material for producing corn plant cells which synthesize the starch of the invention. The expression "wild type corn plant cell", in the context of the present invention, does not comprise corn plant cells of corn mutants of the ae (amylose extender), wx (waxy), du (dull), sh2 (shrunken 2), brittle-1 or brittle-2 genotype or of the double or multiple mutants of these genotypes. The expression "wild type corn plant", in the context of the present invention, means that this is a corn plant which served as starting material for producing corn plants which synthesize the starch of the invention. The expression "wild type corn plant", in the context of the present invention, does not comprise corn mutants of the ae (amylose extender), wx (waxy), du (dull), sh2 (shrunken 2), brittle-1 or brittle-2 genotype or of double or multiple mutants of these genotypes.

Preferably, the expression "wild type corn plant" refers to the corn inbred line A188 which is publicly available, for example via the Maize Genetics Cooperation Stock Center (http://maizecoop.cropsci.uiuc.edu/) at the University of Illinois, Urbana/ Champaign, USA.

The expression "corresponding", in the context of the present invention, means that, in the comparison of a plurality of items, the items in question which are compared with one another are kept under the same conditions. In the context of the present invention the expression "corresponding", in the context of wild type corn plant cell or wild type corn plant means that the plant cells or plants which are compared with one another have been grown under identical cultivation conditions and that they have an identical (cultivation) age.

The expression "increase in the activity of a starch synthase II", in the context of the present invention, means an increase in the expression of endogenous genes which encode proteins having the activity of a starch synthase II and/or an increase of the amount of proteins having the activity of a starch synthase II in the corn plant (cells) and/or preferably an increase of the enzymatic activity of proteins having the activity of a starch synthase II in the corn plant (cells).

The increase in expression can be determined, for example, by measuring the amount of transcripts which encode proteins having the activity of a starch synthase II. The determination can proceed, for example, by Northern Blot analysis or RT-PCR.

The amount of the activity of a protein having the activity of a starch synthase II can be determined, for example, as described in the literature (Nishi et al., 2001, Plant Physiology 127, 459-472). A method for determining the amount of activity of a protein having the activity of a starch synthase II, which method is preferred in the context of the present invention, is described hereinbelow ("determination of SSII activity by means of an activity gel").

Preferably, the corn plant (cells) which synthesize the starch of the invention have an enzymatic activity of starch synthase II which is increased by at least 2 times, preferably 3-10 times, particularly preferably 4-6 times, compared with corresponding wild type corn plant cells or wild type corn plants which are not genetically modified.

In a further embodiment of the present invention, the genetic modification is the introduction of at least one foreign nucleic acid molecule into the genome of the plant cell or into the genome of the plant.

In this context, the expression "genetic modification" means the introduction of at least one foreign nucleic acid molecule into the genome of a corn plant (cell), wherein said introduction of this molecule leads to an increase in the activity of a protein having the activity of a starch synthase II.

By introducing a foreign nucleic acid molecule, the corn plant (cells) are changed in their genetic information. The presence or the expression of at least one foreign nucleic acid molecule leads to a change in phenotype. "Change in phenotype" in this case preferably means a measurable change in one or more functions of the cells. For example, the generically modified corn plant (cells), on account of the presence or on the expression of introduced foreign nucleic acid molecules, they exhibit an increase in the activity of a protein having the activity of a starch synthase II and synthesize a starch according to the invention.

The expression "foreign nucleic acid molecules" is taken to mean, in the context of the present invention, a molecule which either does not occur naturally in corresponding wild type plant cells, or which does not occur naturally in wild type plant cells in the specific spatial arrangement, or which is localized at a site in the genome of the wild type plant cell at which it does not naturally occur. In principle, a foreign nucleic acid molecule can be any desired nucleic acid molecule which, in the plant cell or plant, causes an increase in the activity of a protein having the activity of a starch synthase II.

Preferably, the foreign nucleic acid molecule is a recombinant nucleic acid molecule which comprises various elements, the combination or specific spatial arrangement of which does not occur naturally in plant cells.

The expression "recombinant nucleic acid molecule", in the context of the present invention, is to be taken to mean a nucleic acid molecule which has differing nucleic acid molecules which are not naturally present in a combination as they are present in a recombinant nucleic acid molecule. For instance, the recombinant nucleic acid molecules exhibit, for example, in addition to nucleic acid molecules which encode a protein having the activity of a starch synthase II (for example genomic nucleic acid molecules or cDNAs), have additional nucleic acid sequences which are not naturally present in combination with these nucleic acid molecules. The recombinant nucleic acid molecule has, for example, regulatory sequences (for example promoters, termination signals, enhancers), preferably regulatory sequences which are heterologous with respect to the nucleic acid molecule which encodes the starch synthase II. Heterologous in this context means that the regulatory sequence is not the endogenous regulatory sequence of the starch synthase II gene used itself. In addition, preference is given to regulatory sequences which are active in plant tissue.

Suitable promoters are constitutive promoters such as, for example, the promoter of the 35S RNA of Cauliflower Mosaic Virus (Odell et al., 1985, Nature, 313, 810-812), the ubiquitin promoter from corn (Christensen et at., Plant Mol. Biol. 18, (1992), 675-689), the ubiquitin promoter from rice (Liu et al., Plant Science 165, (2003)), the rice actin promoter (Zhang, et al., Plant Cell 3:1150-1160, 1991), the Cassava Vein Mosaic Virus (CVMV) promoter (Verdaguer et. al., Plant Mol. Biol. 31: 1129-1139), the corn histone H3C4 promoter (US 6,750,378) or the *Cestrum* YLCV promoter (Yellow Leaf Curling Virus; WO 01 73087; Stavolone et al., 2003, Plant Mol. Biol. 53, 703-713).

Particularly preferably these are tissue-specific regulatory sequences which are active in corn tissue, preferably in the endosperm of corn plants. Further endosperm-specific promoters in corn are the promoter of the 10 kD zein gene from corn (Kirihara et al. (1988) Gene 71: 359-370), the 15 kD zein gene from corn (Hoffmann et al. (1987) EMBO J. 6: 3213-3221; Schernthaner et al. (1988) EMBO J. 7: 1249-1253; Williamson et al. (1988) Plant Physiol. 88: 1002-1007), the 27 kd zein gene from corn (Prat et al. (1987) Gene 52: 51-49; Gallardo et al. (1988) Plant Sci. 54: 211-281), the 19 kD zein gene from corn (Marks et al. (1985) J. Biol. Chem. 260: 16451-16459). The relative transcriptional activities of these promoters in corn are described in Kodrzyck et al., (1989), Plant Cell 1, 105-114).

Other promoters which are conceivable in connection with the present invention are the promoter of the sucrose synthase gene (Yang, N.-S. and Russel, D. (1990) Proc. Natl. Acad Sci 87: 4144-4148), of the waxy gene (Unger et al. (1991) Plant Physiol. 96: 124), of the sh 2 gene (Bhave et al. (1990) Plant Cell 2: 581-588, of the bt 2 gene (Bae et al.. (1990) Maydica 35: 317-322). In addition the HMG promoter (also termed wheat glutenin HMWG promoter) from wheat (Colot et al., EMBO J. 6, (1987, 3559-3564; Clarke and Appels, Genome 41, (1998), 865-871), the USP promoter, the phaseolin promoter, promoters of zein genes from corn (Pedersen et al., Cell 29 (1982), 1015-1026; Quatroccio et al., Plant Mol. Biol. 15 (1990), 81-93), the glutelin promoter (Leisy et al., Plant Mol. Biol. 14 (1990), 41-50; Zheng et al., Plant J. 4 (1993), 357-366; Yoshihara et al., FEBS Lett. 383 (1996), 213-218), the globulin promoter (Nakase et al., 1996, Gene 170(2), 223-226) or the prolamine promoter (Qu and Takaiwa, 2004, Plant Biotechnology Journal 2(2), 113-125).

Intron sequences can also be present between the promoter and the coding region. Such intron sequences can lead to stability of expression and to an increased expression in plants (Callis et al., 1987, Genes Devel. 1, 1183-1200; Luehrsen, and Walbot, 1991, Mol. Gen. Genet. 225, 81-93; Rethmeier, et al., 1997; Plant Journal. 12(4):895-899; Rose and Beliakoff, 2000, Plant Physiol. 122 (2), 535-542; Vasil et al., 1989, Plant Physiol. 91, 1575-1579; XU et al., 2003, Science in China Series C Vol. 46 No. 6, 561-569). Suitable intron sequences are, for example, the first intron of the sh1 gene from corn (Maas et al. (1991) Plant. Mol. Biol. 16: 199-207, the first intron of the polyubiquitin gene 1 from corn, the first intron of the EPSPS gene from rice or one of the two first introns of the PAT1 gene from *Arabidopsis,* in addition introns of the Adh-1 or Bz-1 gene from corn (Callis et al. (1987) Genes Dev. 1: 1183-1200), the intron 3 of the corn actin gene (Luehrsen, K. R. and Walbot, V. (1991) Mol. Gen. Genet. 225: 81-93) or of the Adh1 intron 6 (Oard et al. (1989) Plant Cell Rep 8: 156-160).

Methods of producing recombinant nucleic acid molecules are known to those skilled in the art and comprise genetic engineering methods such as, for example, binding nucleic acid molecules by ligation, genetic recombination or denovo synthesis of nucleic acid molecules (see, for example, Sambrok et al., Molecular Cloning, A Laboratory Manual, 3rd edition (2001) Cold Spring Harbour Laboratory Press, Cold Spring Harbour, NY. ISBN: 0879695773, Ausubel et al., Short Protocols in Molecular Biology, John Wiley & Sons; 5th edition (2002), ISBN: 0471250929).

The expression "genome", in the context of the present invention, is to be taken to mean the totality of the hereditary material present in a plant cell. It is known to those skilled in the art that, in addition to the cell nucleus, hereditary material is also present in other compartments (for example plastids, mitochondria).

In a further embodiment, the present invention relates to corn flour comprising the corn starch according to the invention.

In a preferred embodiment, the present invention relates to corn flour comprising the - preferably granular - corn starch according to the invention.

Starch-storage parts of plants can be processed to flours. For production of corn flours, the endosperm-comprising corn kernels are milled and sieved. Starch is a main component of the endosperm. The corn starch of the invention is, in addition to proteins and lipids, the important component of the corn flour of the invention (approximately 65 to 75% by weight of the flour dry weight). The properties of the corn flours of the invention are therefore strongly effected by the corn starch of the invention present in the corn flour.

The expression "corn flour, in the context of the present invention, is to be taken to mean a powder obtained by milling corn kernels, wherein the corn kernels comprise corn plant cells which express a heterologous starch synthase 11. If appropriate, the corn kernels are dried before milling and, after milling, comminuted and/or sieved.

In a further embodiment, the present invention therefore also discloses corn flours, the starch component of which has an amylose content between 15% by weight and 40% by weight, preferably between 18% by weight and 35% by weight, and particularly preferably between 20% by weight and 30% by weight, based on the starch, and the flour has a DSC T-onset temperature between 71.0˚C and 78.0˚C, preferably between 71.5˚C and 77.0˚C, particularly preferably between 72.0˚C and 76˚C.

In a further embodiment, the corn flour disclosed in the invention has a DSC T-peak temperature between 76.0˚C and 85.0˚C, preferably between 77.0˚C and 82.0˚C, particularly preferable between 79.0˚C and 81 ˚C.

It was surprising for those skilled in the art that the DSC T-onset temperature and the DSC T-peak temperature of the corn starch disclosed in the invention or corn flours were considerably increased compared with corresponding starches or flours of wild type corn plants. In particular, because the corn starches and corn flours disclosed in the invention have an amylose content which is in the range of wild type corn plants, which leads to altered processing properties of the corn starches and corn flours disclosed in the invention which are described further below. Such a great increase in DSC T-onset or DSC T-peak temperature of corn starches and corn flours has hitherto only been established in corn starches of amylose extender (ae) mutants having apparent amylose contents increased with respect to wild type corn plants (approximately 40-70% by weight of starch) or in corn starches of amylose extender - waxy (ae wx) double mutants (Sanders et al., Cereal Chemistry 67(6), (1990), 594-602).

In many thermal processes and applications, the use of (granular) corn starches or of corn flours comprising such (granular) corn starches is desirable. Therefore, the high DSC T-onset or T-peak temperature of the corn starches disclosed in the invention or of the corn flours disclosed in the invention is particularly advantageous. On account of this property, the retention of the starch granule structure is ensured even at elevated process temperatures.

In a further embodiment, the corn flours of the invention have a rapidly digestible starch (RDS) content, based on the amount of starch (dry weight) between 10% by weight and 23% by weight, preferably between 12% by weight and 21% by weight, particularly preferably between 14% by weight and 20% by weight.

In a further embodiment, the corn flours of the invention have a rapidly digestible starch (RDS) content based on the amount of starch (dry weight) between 7% by weight and 23% by weight, preferably between 10% by weight and 23% by weight, particularly preferably between 10% by weight and 17% by weight.

In a preferred embodiment, the corn flours of the invention have a rapidly digestible starch (RDS) content based on the amount of starch (dry weight) between 5% by weight and 25% by weight, between 7% by weight and 24% by weight, preferably between 10% by weight and 23% by weight, particularly preferably between 10% by weight and 17% by weight.

In a further embodiment, the corn flours of the invention have a rapidly digestible flour content based on the amount of flour (dry weight) between 9% by weight and 22% by weight, preferably between 10% by weight and 20% by weight, particularly preferably between 12% by weight and 18% by weight.

In a further embodiment, the starch component of the corn flours of the invention has an RS content between 26% by weight and 45% by weight, preferably between 27% by weight and 40% by weight, particularly preferably between 28% by weight and 36% by weight, based on the amount of starch (dry weight).

In a further embodiment, the starch component of the corn flours of the invention has an RS content between 26% by weight and 55% by weight, preferably between 27% by weight and 50% by weight, particularly preferably between 40% by weight and 48% by weight, based on the amount of starch (dry weight).

In a further embodiment, the starch component of the corn flours of the invention has an RS content between 15% by weight and 60% by weight, preferably between 29% by weight and 50% by weight, particularly preferably between 35% by weight and 48% by weight, based on the amount of starch (dry weight).

In a further aspect, the corn flours of the application have a slowly digestible starch (SDS) content based on the amount of starch (dry weight) between 22% by weight and 67% by weight, preferably between 27% by weight and 63% by weight, particularly preferably between 35% by weight and 50% by weight.

In a preferred embodiment, the corn flours of the invention have a slowly digestible starch (SDS) content based on the amount of starch (dry weight) between 20% by weight and 60% by weight, preferably between 30% by weight and 56% by weight, particularly preferably between 40% by weight and 50% by weight.

Determination of the amylose content and of the RS, SDS and RDS content of the starch component of the flour of the invention proceeds in the context of the present invention as already described for the corn starches of the invention, after the corn starch has been isolated from the corn flour Preferably, the corn starch is isolated from the corn flour using the method described herein below "extraction of corn starch".

The RS content of the corn flour of the invention, in the context of the present invention, is determined via the abovementioned method of Englyst et al. (Europ. J. of Clinical Nutrition 46 (Suppl. 2), (1992), S33-50, see, in particular, the following sections from Englyst et al., pages S35-S36: "Reagents, Apparatus, Spectrophotometer"; pages S36-S37, paragraph "Measurement of free glucose (FG)"; page S38, paragraph "Measurement of RDS and SDS"). The "RS content of the corn flour", in the context of the present invention, is designated the fraction of the weighed-out flour sample (dry weight) which is not released as glucose in the method of Englyst et al. after 2 hours. It is therefore given by the following formula:

RS flour in % = 100% – 100% x (glucose released after 2 h in mg/dry weight of flour in mg)

In the context of the present invention, "the content of rapidly digestible flour" is to be taken to mean the fraction of a corn flour which is released as glucose after 20 minutes in the abovementioned method of Englyst et al. for determining the RS content. The report in percent by weight is based in this case on the dry weight of the flour sample. Accordingly, in the context of the present invention the following applies:

Rapidly digestible flour content in % = 100 x glucose released after 20 minutes in mg/ dry weight of flour in mg.

The corn flours of the invention, compared with previously known corn flours having an elevated RS content or reduced content of RDS which, in them is due to a significantly increased amylose content compared with wild type corn flours, are distinguished by a significantly improved processability which results from the fact that the amylose content of the starch component of the flour is scarcely altered compared with wild type corn flours.

The RS amylomaize starches described in the prior art have the disadvantage of poor processing properties, because these starches scarcely gelatinize, have an increased tendency to retrogradation, a low swelling capacity and are poorly soluble in water. For applications in which only gelatinized starches are usable, or the retrogradation tendency is to be decreased (for example to avoid staling processes in bakery products) or a relatively high swelling capacity or a higher solubility are required, the amylomaize starches are therefore either entirely unsuitable, or they must be additionally chemically modified in order to set the desired properties. Compared with these granular corn starches of amylose extender mutants or corn flours comprising such corn starches, the corn starches and corn flours of the invention have the advantage that they have advantageous digestion properties (increased RS content, decreased RDS content) and/or an increased thermal stability and/or, compared with amylomaize starches, significantly increased gelatinization, a decreased tendency to retrodegradation, an increased swelling capacity and/or an increased solubility. As a result, the corn starches and corn flours of the invention are more suitable for those applications in which either only gelatinized starches are usable and/or the tendency to retrogradation is to be decreased and/or in which a high swelling capacity and/or a higher solubility and/or an increased thermal stability is required.

The expression amylose extender (ae) mutants, in the context of the present invention, is taken to mean corn plants (plant cells) which have a mutation of the gene of the starch branching enzyme IIb from corn (abbreviation "BE IIb" or "SBE IIb"), which is also termed the amylose extender gene, wherein this mutation leads to a reduction of the SBE IIb enzyme activity in the endosperm of these corn plants compared with the BE IIb activity in the endosperm of wild type corn plants.

This mutation of the BE IIb in corn plants (plant cells) can lead to no SBE IIb activity being detectable any longer (for example Fisher et al., Plant Physiol. 110, (1996), 611-619, in particular figure 4; Hedman and Boyer, Biochemical Genetics 21 (11/12), (1983), 1217-1222, in particular table 1). A BE IIb protein from corn, in the context of the present invention, is taken to mean a branching enzyme of the isoform IIb which is encoded by what is termed the amylose extender gene (Kim et al., Plant Molecular Biology 38, (1998), 945-956). The branching enzyme (BE) of the isoform IIb ($\alpha$-1,4-glucan: $\alpha$-1,4-glucan 6-glycosyltransferase; E.C. 2.4.1.18) catalyzes a transglycosylation reaction in which $\alpha$-1,4-links of an $\alpha$-1,4-glucan donor are hydrolyzed and the $\alpha$-1,4-glucan chains released in this case are transferred to an $\alpha$-1,4-glucan acceptor chain and are converted in this operation into $\alpha$-1,6-links. In its biochemical properties, the BEIIb protein from corn differs significantly from the BEI protein from corn which are summarized by Fisher et al. (Plant Physiol. 110, (1996), 611-619) in table 1, page 612. For example, the BEI protein branches amylose more rapidly than does the BEIIb protein, whereas the BEIIb protein branches amylopectin at a higher rate than does the BEI protein (Guan and Preiss, Plant Physiol. 102, (1993), 1269-1273). The amino acid sequence of the BEIIb protein differs from the BEIIa protein according to Gao et al. (Plant Physiol. 114, (1997), 69-78) especially by a 49 amino acid long N-terminal extension of the BEIIa protein. The molecular weight of the BEIIa protein determined by means of SDS-PAGE is 89 kD, that of the BEIIb protein somewhat less, that is to say 85 kDa (Fisher et al., Plant Physiol. 110, (1996), 611-619).

An "amylose extender" gene (also "BEIIb" gene) from corn, in the context of the present invention, is taken to mean a gene which encodes a BEIIb protein.

The "amylose extender mutation" can be a dominant mutation of the amylose extender 1 locus which leads to the synthesis of a corn starch that has, compared with wild type corn plants (plant cells), an increased apparent amylose content which is between 50 and 90% by weight. Preferably, the dominant mutation is the Mu-induced allele Ae1-5180 of the amylose extender1-locus (Stinard et al., Plant Cell 5, (1993), 1555-1566).

In addition, the "amylose extender mutation" can be corn plants (plant cells) having homozygotically recessive "amylose extended" genotype that synthesize a corn starch having an apparent amylose content of approximately 50-90% by weight. The amylose extender 1-(ae1)-locus comprises the structural gene which encodes the SBE IIb protein (Hedman and Boyer, Biochemical genetics 20 (5/6), (1982), 483-492).

Amylose extender (ae) corn mutants have been described, for example, in Vineyard and Bear (Maize Genet Coop Newsletter 26: 5 (1952), who described the reference allele ae1-Ref and also in Moore and Creech (Genetics 70, (1972), 611-619), Garwood et al. (Cereal Chemistry 53(3), (1976), 355-364) and Hedman and Boyer (Biochemical Genetics 21 (11/12), (1983), 1217-1222).

The corn starches/corn flours of the invention in addition have the advantage of a decreased fraction of rapidly digestible flour or starch, which is particularly advantageous, since rapid release of relatively large amounts of glucose and its absorption via the small intestine epithelium lead to an abrupt increase in the blood sugar level. In consequence therefore, there is an excretion of insulin (insulin response). The continuous consumption of foods having a high glycemic charge and the associated insulin excretion is under suspicion as a risk factor in the development of diseases such as high blood pressure, overweight, heart diseases and diabetes type II.

In a further embodiment, the present invention discloses a corn flour which comprises a - preferably granular - corn starch which, in addition to an amylose content between 15% by weight and 40% by weight, preferably between 18% by weight and 35% by weight, and particularly preferably between 20% by weight and 30% by weight, in addition optionally has

a) an RS content between 26% by weight and 45% by weight, preferably between 27% by weight and 40% by weight, particularly preferably between 30% by weight and 36% by weight and/or

b) a rapidly digestible starch (RDS) content, based on the amount of starch (dry weight), between 10% by weight and 23% by weight, preferably between 12% by weight and 21% by weight, particularly preferably between 14% by weight and 20% by weight and/or

c) a DSC T-onset temperature between 70.5˚C and 77.5˚C, preferably between 71.0˚C and 76.5˚C, particularly preferably between 71.5˚C and 75.5˚C and/or

d) a DSC T-peak temperature between 75.5˚C and 64.5C. preferably between 76.5˚C and 81.5˚C, particularly preferably between 77.5˚C and 80.5%C

In a further aspect, the present application discloses a corn flour that comprises a - preferably granular - corn starch which, in addition to an amylose content between 15% by weight and 40% by weight, preferably between 18% by weight and 35% by weight, and particularly preferably between 20% by weight and 30% by weight, optionally additional has

a) an RS content between 26% by weight and 55% by weight, preferably between 27% by weight and 50% by weight, particularly preferably between 40% by weight and 48% by weight, and/or

b) a rapidly digestible starch (RDS) content based on the amount of starch (dry weight) between 5% by weight and 25% by weight, between 7% by weight and 23% by weight, preferably between 10% by weight and 23% by weight, particularly preferably between 10% by weight and 17% by weight, and/or

c) a slowly digestible starch (SDS) content based on the amount of starch (dry weight) between 22% by weight and 67% by weight, preferably between 27% by weight and 63% by weight, particularly preferably between 35% by weight and 50% by weight, and/or

d) a DSC T-onset temperature between 67.5˚C and 75.0˚C, preferably between 68.0˚C and 74.5˚C, particularly preferably between 68.5˚C and 74.0˚C, and/or

e) a DSC T-peak temperature between 75.5˚C and 84.5˚C, preferably between 76.5˚C and 81.5˚C, particularly preferably between 77.5˚C and 80.5˚C.

In a further preferred embodiment, the present invention discloses a corn flour which comprises a - preferably granular - corn starch which, in addition to an amylose content between 15% by weight and 40% by weight, preferably between 18% by weight and 35% by weight, and particularly preferably between 20% by weight and 30% by weight, optionally additionally has

a) an RS content between 15% by weight and 60% by weight, preferably between 29% by weight and 50% by weight, particularly preferably between 35% by weight and 48% by weight, and/or

b) a rapidly digestible starch (RDS) content based on the amount of starch (dry weight) between 7% by weight and 25% by weight, preferably between 10% by weight and 23% by weight, particularly preferably between 10% by weight and 17% by weight, and/or

c) a slowly digestible starch (SDS) content based on the amount of starch (dry weight) between 20% by weight and 60% by weight, preferably between 30% by weight and 56% by weight, particularly preferably between 40% by weight and 50% by weight, and/or

d) a DSC T-onset temperature between 67.5˚C and 75.0˚C, preferably between 68.0˚C and 74.5˚C, particularly preferably between 68.5˚C and 74.0˚C and/or

e) a DSC T-peak temperature between 75.5˚C and 84.5˚C, preferably between 76.5˚C and 81.5˚C, particularly preferably between 77.5˚C and 80.5˚C.

The present invention further discloses a process for producing the flours of the invention comprising the step of milling at least one corn plant which expresses a heterologous starch synthase II.

In a further embodiment of the process for producing the flours of the invention, corn kernels are ground which comprise corn plant cells which express a heterologous starch synthase II.

Preferably, the process for producing flours also comprises the step of harvesting the corn plants or corn kernels of these corn plants before milling, preferably washing the corn plants or the corn kernels before milling, and in addition, the step of cultivating the corn plants before harvesting.

In a further disclosure, the process of the invention for producing flours comprises processing the corn plants or corn kernels, the plant cells of which express a heterologous starch synthase II, before milling.

The processing in this case can be, for example, a heat treatment and/or a drying. The comminution of corn plants, of starch-storage parts or corn kernels of such corn plants before milling can likewise be a processing within the meaning of the present invention. The removal of plant tissue such as, for example, husks of kernels, before milling is also a processing before milling within the meaning of the present invention.

In a further disclosure, the process for producing flours comprises after milling a processing of the milling material.

The milling material in this case can be sieved, for example, after milling, in order to produce various flour types, for example.

In a further embodiment, the present invention relates to the use of corn flour of the invention for producing a food, which has a reduced glycemic index compared with the glycemic index of a food which comprises flour from corresponding wild type plants.

In a further embodiment, the present invention relates to the use of corn flour of the invention as a prebiotic.

In a further embodiment, the present invention relates to a composition comprising the corn starch of the invention and at least one food additive.

In a further embodiment, the present invention relates to a composition comprising the corn flour of the invention and at least one food additive.

As food additive, in the context of the present invention, use shall be made, for example, of vitamins (for example vitamin A, B1, B2, B3, B5, B6, B9, B12, C, D, E, F, K), provitamins, antioxidants, trace elements (for example chromium, iron, fluorine, iodine, cobalt, copper, manganese, molybdenum, selenium, vanadium, zinc), major elements (for example calcium, chlorine, potassium, magnesium, phosphorus, sulfur, sodium), flavorings, dyes, oils, fats, fatty acids, in particular (poly)unsaturated fatty acids, essential fatty acids, carbohydrates (for example starches, galactooligosaccharides, gentiobiose, tagatose), dietary fibers (for example cellulose, hemicellulose, pectin, ligin), prebiotics (for example oligofructose, oligosaccharides, chitosan, beta-glucans, arabinogalactan), probiotics (for example bifidobacteria, lactic acid bacteria such as, for example, the genus Lactobacillus), i.e. non-pathogenic microorganisms which are added to the food alive or in spore form and which can beneficially effect the intestinal flora.

The compositions of the invention can be produced, for example, by simple mixing.

In a further embodiment, the present invention relates to a food comprising the corn starch of the invention, wherein the food has a reduced glycemic index compared with the glycemic index of a food which comprises starch from corresponding wild type corn plants

In a further embodiment, the present invention relates to a food comprising the corn flour of the invention, wherein the food has a reduced glycemic index compared with the glycemic index of a food which comprises flour from corresponding wild type corn plants.

In a further embodiment, the present invention relates to a food comprising the composition of the invention.

Typical foods which can be produced using the corn starch of the invention, the corn flour of the invention, or the composition of the invention, are, for example, tortillas, tortilla chips, bakery products (for example bread, corn bread, rolls, biscuits, cakes, waffles, muffins, tacos), pancakes, pizza, polenta, pasta (for example noodles), "cornmeal mush" (USA), "porridge" (GB), stews, sauces, corn flour pudding, milk products (for example yoghurt, quark, ice cream), puddings, spreads (for example butter, margarine), drinks, drink powders, prepared dishes, (breakfast) cereals, enchiladas, sausage products, meat products, baby food, ketchup, mayonnaise, barbecue sauces and others.

**Material and methods**

In the examples, the following methods were used. These methods can be used to carry out the process of the invention, they are specific embodiments of the present invention, but do not restrict the present invention to these methods.

**1) Plant material and cultivation**

Corn plants: *Zea mays,* variety A188

The corn plants were cultivated in the greenhouse under the following conditions:

| | |
|---|---|
| **Substrate:** | Special mixture for seeding<br>80% light peat<br>20% dark peat<br>100 kg/$m^3$ sand<br>40 kg/$m^3$ moist clay<br>Structure: fine<br>pH: 5.3-6.1<br>Base fertilization: 2 kg/$m^3$ 12-12-17 (+2) and 100g/$m^3$<br>Radigen (Theraflor GmbH; Iserlohn; Germany) |
| **Pots:** | 10 l containers |
| **Planting density:** | Max. 6 plants/$m^2$ |
| **Fertilization:** | 1 TAB Plantosan 4 g (20-10-15+6) in the 4-leaf stage<br>1 TAB Plantosan after a further 3 weeks |
| **Temperature:** | Day 22-25˚C/night 16˚C |

| | |
|---|---|
| **Light:** | 18 hours, 350-400µEinstein/s/m |
| **Relative humidity**: | 50% rh. |
| **Plant protection measures:** | as required insecticides: for example: Vertimec (Syngenta), Confidor (Bayer) |

**2) Origin of the sequences and constructs used for the transformation**

For transformation of corn, use was made of the sequence Ta_SSIIa from wheat. Isolation and cloning were performed as described in international patent application WO 97/45545 (under the then designation "pTaSS1"). The transformation vector pJH77 used is described in example 1.

**3) Transformation and regeneration of corn plants**

Corn plants were transformed and regenerated by the method described by Ishida et al. (1996 Nature Biotechnology Vol. 14: 745-750).

**4) Processing of corn kernels**

For generation of sufficient amounts of analysis material, corn plants were grown under greenhouse conditions and after reaching complete ripeness, the cobs were harvested. For further drying, the ripe (i.e. fully developed) corn cobs were stored for 3-7 days at 37°C.

Subsequently, the kernels were taken off from the cobs. These served as starting material for analysis of the whole kernel, such as, for example, kernel weight.

**5) Analysis of the level of expression of starch synthase II by RTPCR**

The expression of starch synthase IIa from wheat in corn was studied by RTPCR. For this, for each independent transgenic event, four ripe corn kernels were studied. For homogenization, the corn kernels were shaken in the 1.5 ml Eppendorf vessel with a 4.5 mm steel ball in a Retsch mill (model MM300) for 30 seconds at a frequency of 30 hertz. Subsequently the RNA was isolated by means of a "Plant RNAeasy" Quiagen Kit, according to the manufacturer's instructions (Plant RNAeasy, Quiagen, Germany). On the basis of a PCR with a previous reverse transcriptase step, the relative expression of the SS2 gene in the transgenic plants can be demonstrated.

Primer sequences:

| | |
|---|---|
| 1. Ta_SS2-F4: | 5'- gAA gAA gCT CCA AAg CCA AA -3' (SEQ ID No.3) |
| 2. Ta_SS2-R4: | 5'- ggC TCC TCg AAA CCA ATg TA -3' (SEQ ID No.4) |
| 3. Ta SS2-FAM | 5'- TCT TgA AAT CCC AAA ggT CTT CTT gTA -3' (SEQ ID No.5) |
| 1a.18S.F1: | 5'- gTC ATC AgC TCg CgT TgA CT-3' (SEQ ID No.6) |
| 2a. 18S-R1: | 5'- TCA ATC ggT Agg AgC gAC g-3' (SEQ ID No.7) |
| 3a. 18S-VIC : | 5'- ACG TCC CTg CCC TTT gTA CAC ACC gC-3' (SEQ ID No.8) |

PCR reaction batch (QUIAGEN OneStep RT-PCR Kit):

Final concentrations:

- RNA (50-500 ng)
- 1 x Quiagen buffer
- 1 x Q-solution
- 0.4 mM dNTPs

- 0.5 mM MgCl2
- 40 SYBR GREEN
- 0.11 μM TagMan probe
- 0.3 μM Forward Primer
- 0.3 μM Reverse Primer
- 1.2 μl Quiagen Enzyme mix

Conditions (PE Applied Biosystems ABI PRISM 7700):

| | | |
|---|---|---|
| 30 min | 60°C | |
| 15 min | 95°C | |
| 30 sec | 94°C | 35 cycles |
| 30 sec | 60°C | |
| 60 sec | 72°C | |

The PCR is quantified on the basis of calculation of the fluorescence threshold value, what is termed the threshold cycle or CT value. The CT value is that PCR cycle in which the reporter fluorescence significantly exceeds the background fluorescence. At the start of the PCR reaction, only the base fluorescence or background fluorescence is measured, since the reporter fluorescence is usually not detectable owing to the low template concentration in the reaction vessel during the first PCR cycles. Quantification of the amount of DNA is not based on absolute amounts of PCR product, but on the kinetics of the PCR reaction. The CT value is taken as guideline of this, since at this time point the amplification is exponential. In parallel thereto, in each PCR run, known amounts of template are amplified, so that it is possible to compare what amount of template is obtained at what CT value. A standard curve can be prepared therefrom on the basis of which the template concentration can be concluded.

For calculation of the levels of expression, the following formula applies:

$$\frac{X_{N,q}}{X_{N,cb}} = (1+E)^{-\Delta\Delta Ct} = 2^{-\Delta\Delta Ct} \text{ (for } E = 1)$$

$X_N$ = normalized amount of target
E = efficiency of the PCR

$X_N$ = normalized amount of target
$\Delta C_T$ = difference between $C_T$ values of target gene and reference

This gives: $X_N = K(1 + E)^{-\Delta C_T}$

For each sample q which is compared with the calibrator cb, the following applies:

$$X_{N,q} = K(1 + E)^{-\Delta C_{T,q}}$$

$$X_{N,cb} = K(1 + E)^{-\Delta C_{T,cb}}$$

### 6) Determination of SSII activity by means of an activity gel

The various starch synthase activities in unripe corn kernels were detected by means of activity gels (zymograms), in which protein extracts are separated in a polyacrylamide gel under native conditions and are subsequently incubated with appropriate substrates. The reaction product formed (starch) was stained with Lugol's solution (2% (w/v) KI; 0.2% (w/v) 12) in the gel.

Individual unripe corn kernels (approximately 15 days after blossom, measured from the day of start of blossom) were shock-frozen in liquid nitrogen and homogenized in 150-200 μl of cold extraction buffer (50 mM Tris/HCl pH 7.6, 2.5 mM EDTA, 2 mM DTT, 4 mM PMSF, 0.1% (w/v) glycogen, 10% (v/v) glycerol). After centrifugation (15 min, 13000 g, 4°C), the clear supernatant was transferred to a fresh reaction vessel and one aliquot of the extract was used to determine the protein content according to Bradford (1976, Anal Biochem 72: 248-254).

The protein extracts were separated by means of a continuous 7.5% polyacrylamide gel (7.5% AA/BAA 37.5: 1; 25 mM Tris/HCl pH 7.6, 192 mM glycine, 0.1% (w/v) APS, 0.05% (v/v) TEMED) using one time concentrated running buffer (25 mM Tris/HCl, 192 mM glycine). Before the gels are loaded there is a preliminary run to remove free radicals for 30 minutes at 8mA and 4°C. For each sample, 30 μg of protein were applied and the electrophoresis was carried out for 2-2.5 hours at 4°C. Thereafter, the gels were incubated overnight at room temperature with constant shaking in 15 ml of incubation buffer (0.5M sodium citrate pH 7.0, 25mM potassium acetate, 2mM EDTA, 2mM DTT, 0.1% (w/v) amylopectin, 50mM tricine/NaOH pH 8.5, 1mM ADP-glucose). The starch formed was stained using Lugol's solution.

To determine how many times the activity of a protein having the activity of a starch synthase II has increased compared with corresponding wild type plants which have not been genetically modified, protein extracts of the genetically modified lines were in each case sequentially diluted and separated by electrophoresis in accordance with the above described method. The further steps were performed as described above. After the zymograms were stained with Lugol's solution, optical comparison was carried out of the intensity of the stained products produced by a protein having the activity of a starch synthase II (indicated in fig. 2 by an arrow) for the various dilutions of the protein extracts of genetically modified plants with the relevant products of the undiluted wild type protein extract. Since the intensity of staining of the products is directly correlated with the activity of a protein having the activity of a starch synthase II, bands of the products having the same intensities have the same activity. If the bands of the product of a protein having the activity of a starch synthase II in the diluted protein extract has the same intensity as the relevant band of the product from corresponding undiluted protein extract from wild type plants, the dilution factor corresponds to the degree of increase of activity in the relevant genetically modified plant.

### 7) Extraction of corn starch

Corn starch was extracted on the basis of the method described by the corn refiners association (http: //www.corn.org/) for wet starch extraction. 10-50 g of corn kernels were weighed out and to disintegrate the protein matrix were incubated in an excess with 0.2% strength sulfurous acid for 3 days at 50°C. The kernels were then washed with water and briefly dried. Comminution is performed in a Retsch ultracentrifuge mill ZM100 using a 2 mm sieve. The comminuted material was transferred to a glass beaker, admixed with 20% strength NaCl solution and allowed to stand for at least 30 min. In this case the starch sediments and the lipid bodies float. The upper layer (germ) was poured off and the sediment resuspended in a residual supernatant. Subsequently, the starch was further purified by a plurality of sieving steps. First using a 500 μm test sieve (DIN 4188), subsequently using a 200 μm Retsch analysis sieve (DIN 4188) and finally the sample was passed through a 125 μm sieve (Iso 3310-1) and rinsed with NaCl (2-3 l) using a pressure spray system until the drops under the sieve no longer comprised starch. This prepurified starch was sedimented overnight at room temperature and subsequently the supernatant down to approximately 5 mm above the sediment was poured off. The starch was transferred to a centrifuge beaker and centrifuged in a Heraeus Varifuge at room temperature with 3500 rpm for 10 min. Subsequently, the upper starch-protein layer (mostly different in color) was scraped off and discarded.

A plurality of washing steps followed hereinafter, first using 0.2M sodium acetate pH 4.6 (centrifugation see above 5 min), wherein the upper starch-protein layer was again scraped off. Subsequently thereto, a digest was made up in 0.2M sodium acetate pH 4.6 comprising 1% Bromelain and 1% Pepsin in each case and was incubated at 37°C for one hour on the rotator (vertical shaker). Subsequently the batch was centrifuged (see above) and the supernatant discarded. The upper starch-protein layer was again discarded and the pellet resuspended with mains water and centrifuged (see above 3000 rpm). A renewed mechanical separation followed of the protein layer which was situated on the pellet and generally clearly delimited. Four further wash steps with water followed, as described above. Subsequently, the pellet was washed four times with 80% technical ethanol and centrifuged (see above 3000 rpm). Finally, the batch was washed once with acetone in order to defat the starch and dried for two days at room temperature in a fume cupboard.

**8) Preparation of corn flour/corn starch for studying the amylopectin side chain distribution by means of high pressure anion-exchange chromatography**

Per sample, 10 mg of corn flour or corn starch were weighed out into a 2 ml Eppendorf cup and admixed with 250 µl of 90% (v/v) DMSO. After the sample was dissolved with shaking at 60°C, 375 µl of water were added and the batch was incubated for one hour at 95°C. To 200 µl of the batch, 300 µl of 16.7 mM sodium acetate pH 3.5 and also 0.5 U of isoamylase from Pseudomonas sp. (Megazyme; Bray, Ireland) were added. After incubation for 24 hours at 37°C, a further 0.5 U of isoamylase was added and the incubation was continued for a further 24 hours.

For the chromatography, 100 µl of the batch was diluted 1:5 with water and subsequently filtered through Ultrafree-MC Filtertubes (Millipore). About 90 µl of the filtrate was injected.

Chromatography method:

| | | |
|---|---|---|
| HPLC unit: | GP 50 Dionex Gradient Pump | |
| | ED 50 | Dionex Electrochem. Detector/ PAD |
| | AS 50 | Autosampler |
| | column oven | |
| Column: | Dionex CarboPac PA 100 4 x 250 mm (P/N 046110) | |
| | with Guard Column PA 100 4 x 50 mm (P/N 046115) | |

Equipment configuration:

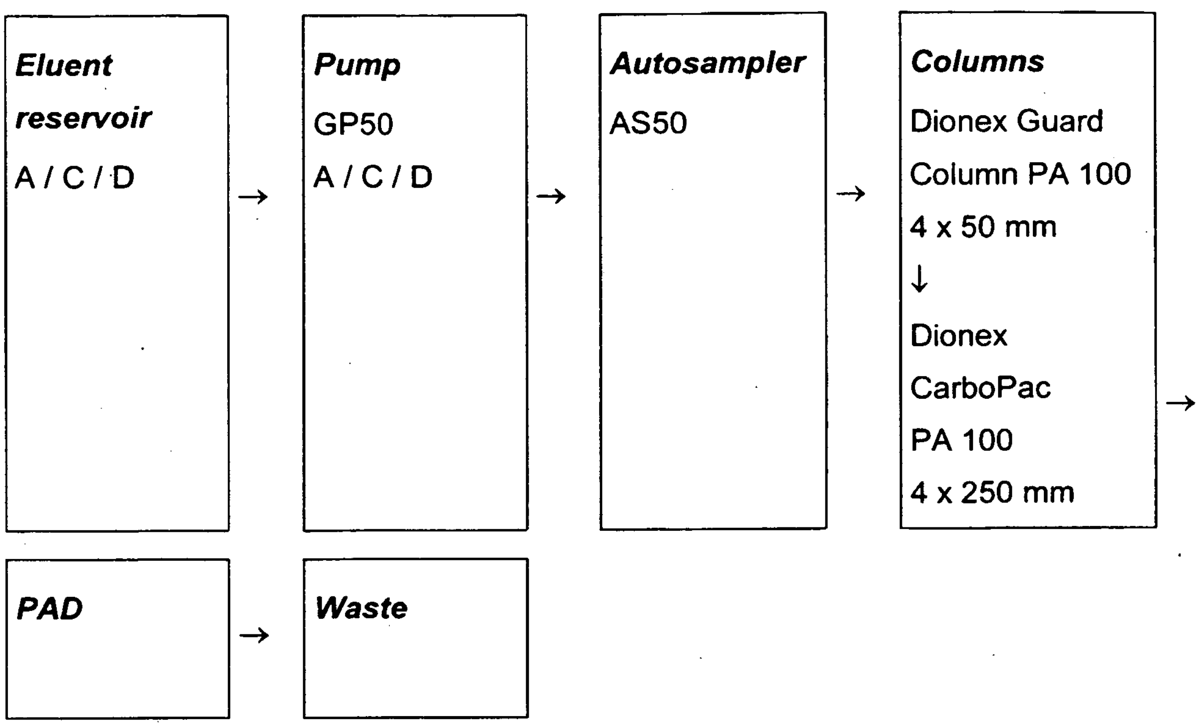


**HPAEC program:**

Pressure.LowerLimit = 50
Pressure.UpperLimit = 3500
%A.Equate ="NaOH 0.15 M"

```
%B.Equate ="NaOAc 1.0 M"
%C.Equate = "NaOAc 1.0 M in NaOH 0.15 M"
%D.Equate = "Millipore Water"
ECD.Data_Collection_Rate = 1.0
Waveform Time = 0.00, Potential = 0.05
Waveform Time = 0.20, Potential = 0.05, Integration = Begin
Waveform Time = 0.40, Potential = 0.05, Integration = End
Waveform Time = 0.41, Potential = 0.75
Waveform Time = 0.60, Potential = 0.75
Waveform Time = 0.61, Potential = -0.15
Waveform Time = 1.00, Potential = -0.15
Cell = On
Flush Volume = 500
Wait FlushState
NeedleHeight = 2
CutSegmentVolume = 10
SyringeSpeed = 4;
Cycle = 0
Wait For Temperature = False
Wait SampleReady

                0.000 Flow =    1.00
                                %B = 0.0
                                %C = 0.0
                                %D = 0.0
                                Curve = 5
                                Load
                                Inject
                                Wait
                                ECD.Autozero
                                ECD_1.AcqOn
                                Flow = 1.00
                                %B = 0.0
                                %C = 0.0
                                %D = 0.0
                                Curve = 5
                5.000 Flow =    1.00
                                %B = 11.0
                                %C = 0.0
                                %D = 0.0
                                Curve = 5
                                Flow = 1.00
                                %B = 11.0
                                %C = 0.0
                                %D = 0.0
                                Curve = 4
                  130.000       Flow = 1.00
                                %B = 35.0
                                %C = 0.0
                                %D = 0.0
                                Curve = 4
                  132.000       Flow = 1.00
                                %B = 0.0
                                %C = 100.0
```

(continued)

| | |
|---|---|
| | %D = 0.0 |
| | Curve = 5 |
| 133.000 | Flow = 1.00 |
| | %B = 0.0 |
| | %C = 100.0 |
| | %D = 0.0 |
| | Curve = 5 |
| 142.000 | Flow = 1.00 |
| | %B = 0.0 |
| | %C = 0.0 |
| | %D = 0.0 |
| | Curve = 5 |
| 143.000 | Flow = 1.00 |
| | %B = 0.0 |
| | %C = 0.0 |
| | %D = 95.0 |
| | Curve = 5 |
| 152.000 | Flow = 1.00 |
| | %B = 0.0 |
| | %C = 0.0 |
| | %D = 95.0 |
| | Curve = 5 |
| ECD_1.AcqOff | |
| End | |

The data were evaluated using a Dionex Chromeleon v6.60 (Dionex Corporation, Sunnyvale, California, USA). The handbook "Tutorial and User Manual" of Version 6.60, March 2004, can be obtained via Dionex, or can be downloaded via the homepage (http://www.dionex.com).

For comparison of the chromatograms with one another, for each chromatogram the identified peaks of the different maltooligasaccharides were mean-value normalized (sum of all peak areas = 1). Evaluation was performed on the basis of "force common baseline", as described in Dionex Chromeleon v.6.60 for "log baseline". In this case the log baseline is set just before the first side chain peak and up to the last evaluable peak of the shortest chromatogram of a measurement passage, from this the last evaluable peak for all chromatograms is calculated.

**9) Thermal analysis of corn flour/corn starch by means of differential scanning calorimetry (DSC)**

Weighed samples of about 10 mg (dry weight) of corn flour or corn starch were placed in stainless steel pans (Perkin Elmer, "Large Volume Stainless Steel Pans" [03190218], volume 60 µl) with an excess, preferably a 2-fold excess of bidistilled water (preferably 20 µl) and hermetically sealed using a press. The sample was heated from 20˚C to 150˚C in a Diamond DSC apparatus (Perkin Elmer) at a heating rate of 10˚C/min. In this method an empty sealed stainless steel pan was used as reference. The system was calibrated with known amounts of indium.

The data analysis was carried out using the Pyris software program (Perkin Elmer, Version 7.0). Evaluable raw data were further processed by analysis of the individual peaks of the first order phase transitions to T-onset (˚C), T-peak (˚C), T-end (˚C) and dH (J/g) (the standard in this case is the straight baseline).

DSC T-onset is characterized in this case as the intersection between the extrapolation of the baseline and the tangent to the ascending flank of the peak through the inflection point. It characterizes the start of the phase transition.

The maximum temperature DSC T-peak is termed the maximum temperature at which the DSC curve has reached a maximum (i.e. the temperature at which the first derivative of the curve is zero).

In the case of the function used in Pyris (calculated peak area) a start temperature and a final temperature are entered by hand for the baseline fit.

**10) Determination of the apparent amylose content**

The apparent amylose content is determined on the basis of the method of Juliano (1971, Cereal Science Today 16 (10): 334-340).

For each sample, 50 mg of corn flour were weighed out twice into 100 ml Erlenmeyer flasks and moistened sequentially with 1 ml of 95% ethanol and 9 ml of 1M NaOH.

In parallel, to establish a standard curve, flasks comprising defined amounts of pure amylose were treated in the same manner as the flour samples. For this purpose, for example, use can also be made of a native corn starch from Sigma-Aldrich (order No. S4126, batch number: #015K0144) which, according to the manufacturer's specifications, has an amylose content of 27% by weight and an amylopectin content of 73% by weight.

The flasks, for thorough mixing, were briefly swirled and subsequently incubated for 20 minutes in a boiling water bath with gentle shaking. After cooling for 5-10 minutes at RT, the volume was made up to 100 ml with water.

One aliquot of 100 µl was admixed with 1 ml of measurement solution (10mM acetic acid, 0.004% (w/v) 12; 0.04% (w/v) KI), mixed well and the absorption was determined at 620 nm against a corresponding blank. The amylose content was calculated using the amylose standards which are used to establish a calibration curve.

**11) Analysis of corn starch by means of a Rapid Visco Analyser (RVA)**

The principle of this analysis is based on the fact that a suspension of water and corn starch is subjected to a defined temperature and shearing protocol and during this the viscosity of the suspension is continuously recorded. The measuring instrument used is an RVA Super3 from Newport Scientific (Macclesfield, UK) with the corresponding software "Thermocline for Windows", Version 2.3.

For the analysis, 2.5 g of corn starch (weighed as pure dry weight of the sample material, corrected to 0% moisture) were weighed out into a measurement vessel, admixed with 25 ml of water and the measuring instrument was plugged into the apparatus after insertion of a stirrer.

The following temperature and shearing profile was applied:

| Time | Type | Value |
|---|---|---|
| 00:00:00 | Temp | 50°C |
| 00:00:00 | Speed | 960 rpm |
| 00:00:10 | Speed | 160 rpm |
| 00:01:00 | Temp | 50°C |
| 00:04:45 | Temp | 95°C |
| 00:07:15 | Temp | 95°C |
| 00:11:00 | Temp | 50°C |
| 00:17:00 | End of test | |

After termination of the measurement, the following parameters were determined:

Peak viscosity (highest viscosity between 2 and 7 minutes of measuring time)
Trough viscosity (lowest viscosity between 7 and 12 minutes of measuring time)
Final viscosity (viscosity at the end of the measurement)
Breakdown = peak - trough
Setback = final - trough
Pasting temperature (temperature at which the viscosity changes by more than 50cP in a time interval of 0.5 minutes)
Peak time (time at which the peak viscosity is achieved)

**12) Determination of the content of phosphate in the C6 position (C6-P content)**

In the starch, positions C3 and C6 of the glucose units can be phosphorylated. For determination of the C6-P content of the starch (modified according to Nielsen et al., 1994, Plant Physiol. 105: 111-117) 50 mg of corn flour/starch were hydrolyzed in 500 µl of 0.7M HCl for 4 h at 95°C with constant shaking. Subsequently, the batches were centrifuged for 10 min at 15 500 g and the supernatants were freed from suspended matter and haze by means of a filter membrane

(0.45 μM). 20 μl of the clear hydrolysate were mixed with 180 μl of imidazole buffer (300 mM imidazole, pH 7.4; 7.5 mM MgCl2, 1 mM EDTA and 0.4 mM NADP). The measurement was carried out in the photometer at 340 nm. After determination of the base absorption, the enzyme reaction was started by adding 2 units of glucose-6-phosphate dehydrogenase (from *Leuconostoc mesenteroides,* Boehringer Mannheim). The change in absorption is based on an equimolar reaction of glucose-6-phosphate and NADP to give 6-phosphogluconate and NADPH, wherein the formation of NADPH is determined at the abovementioned wavelength. The reaction was followed until a plateau was reached. The result of this measurement gives the content of glucose-6-phosphate in the hydrolysate. From the identical hydrolysate, on the basis of the content of glucose released, the degree of hydrolysis was determined. This is used in order to relate to the content of glucose-6-phosphate to the fraction of hydrolyzed starch from the fresh weight. For this, 10 μl of hydrolysate were neutralized with 10 μl of 0.7M NaOH and subsequently diluted 1:100 with water. 4 μl of this dilution were admixed with 196 μl of measurement buffer (100mM imidazole pH 6.9; 5 mM MgCl2, 1 mM ATP, 0.4 mM NADP) and used for determination of the base absorption. The reaction was started by addition of 2 μl of enzyme mix (hexokinase 1:10; glucose-6-phosphate dehydrogenase from yeast 1:10 in measurement buffer) and followed at 340 nm to the plateau. The principle of measurement corresponds to that of the first reaction.

The result of this measurement gives the amount of glucose (in mg) which was released from the starch present in the starting material in the course of the hydrolysis.

Subsequently, the results of both measurements were related to express the content of glucose-6-phosphate per mg of hydrolyzed starch. Contrary to a relation of the amount of glucose-6-phosphate to the fresh weight of the sample, this calculation only relates the amount of glucose-6-phosphate to the part of the starch which was completely hydrolyzed to glucose, and therefore is also to be considered as source for glucose-6-phosphate.

**13) Determination of the resistant starch fraction (digestibility)**

The resistant starch fraction is determined according to the method described in Englyst et al. (Europ. J. of Clinical Nutrition 46 (Suppl. 2), (1992), S33-50)) (see, in particular, the following sections from Englyst et al., pages S35-S36: "Reagents, Apparatus, Spectrophotometer"; pages S36-S37, paragraph "Measurement of free glucose (FG)"; page S38, paragraph "Measurement of RDS and SDS").

The method of Englyst et al. can alternatively be carried out as described by Zhang et al. (Biomacromolecules 7, (2006), 3252-3258, in particular page 3253: Methods. Enzymatic Starch Hydrolysis).

On laboratory scale, the method of Englyst et al. can be carried out in the following manner using corn starch or corn flour.

For production of the enzyme solution, 1.2 g of pancreatin (Merck) are extracted in 8 ml of water for 10 minutes at 37˚C. After centrifugation (10’, 3000 rpm; RT), 5.4 ml of the supernatant are mixed with 84U of amyloglucosidase (Sigma-Aldrich, Taufkirchen) and made up with water to a final volume of 7 ml.

In parallel, 10 mg (dry weight) of corn flour or corn starch per sample in a 2 ml reaction vessel are admixed with 0.75 ml of sodium acetate buffer (0.1 M sodium acetate pH 5.2; 4 mM $CaCl_2$) and incubated at 37˚C for 5 minutes to warm the batch.

The starch digestion is started by adding in each case 0.25 ml of enzyme solution per batch. The control used is a batch to which water is added instead of enzyme solution. After 20, 60 and 120 minutes, aliquots of 100 μl are taken and placed directly into four times the volume of ethanol, as a result of which the enzymes are inactivated. This dilution is used to measure the glucose content.

For this, 2 μl of diluted sample are mixed with 200 μl of measurement buffer (100 mM imidazole/HCl pH 6.9, 5 mM $MgCl_2$, 1 mM ATP, 2 mM NADP) and the absorption of the sample is determined at 340 nm. The reaction of the glucose is started by addition of 2 μl of enzyme mix (10 μl of hexokinase, 10 μl of glucose-6-phosphate dehydrogenase, 80 μl of measurement buffer) and the equimolar conversion of NADP to NADPH at 340 nm is followed until a plateau is reached. The amounts of glucose determined are related to the amount weighed out and give the fraction of the sample which was released as glucose after the corresponding period.

The examples hereinafter illustrate the above described invention.

**Example 1: Production of the vector pJH77 for expression of a starch synthase II from wheat in corn**

The vector pJH77 (see figure 1) has the genetic elements described in table 1:

Table 1: Description of the genetic elements of JH77

| Nt positions | Orientation | Origin |
|---|---|---|
| 6600-6623 | | **RB**: Right hand border of T-DNA from *Agrobacterium tumefaciens (Zambryski,* 1988) |
| 6624-6909 | | Remaining TL-DNA of pTiAch5, which flanks the right hand border (Gielen *et al.,* 1984) |
| 6910-7285 | Anticlockwise | **3' nos**: Sequence comprising the 3' untranslated region of the nopalin synthase gene of T-DNA of the plasmid pTiT37 (Depicker *et al.,* 1982) |
| 7286-9685 | Anticlockwise | ***ss2aTa***: Coding sequence of the starch synthase isoform 2a gene (ss2a) from *Triticum aestivum* (wheat) (SEQ ID No.1) |
| 9686-10437 | Anticlockwise | **intron1 ubi1 Zm**: First intron of the ubiquitin-1 gene *(ubi1)* from *Zea mays* (Christensen *et al.,* 1992). |
| 10438-11478 | Anticlockwise | **PglobulinOs**: Sequence comprising the promoter region of the globulin gene from Oryza sativa (rice) (Hwang *et al.* (2002)) |
| 11479-13261 | Clockwise | **Pact1Os**: Sequence comprising the promoter region of the actin 1 gene from Oryza *sativa* (rice) (McElroy et al., 1990) |
| 13262-13739 | Clockwise | **intron1 act1 Os**: First intron of the actin 1 gene from *Oryza sativa* (rice) (McElroy et al., 1990) |
| 13740-14291 | Clockwise | ***bar*** Coding sequence of the phosphinothricin acetyltransferase gene from *Streptomyces hygroscopicus* (Thompson *et al.* (1987)) |
| 14292-14561 | Clockwise | **3' nos**: Sequence comprising the 3' untranslated region of the nopalin synthase gene of T-DNA of the plasmid pTiT37 (Depicker *et al.,* 1982) |
| 14562-296 | | Remaining TL-DNA of pTiAch5 which flanks the left hand border (Gielen *et al.,* 1984) |
| 297-320 | | **LB**: Left hand border of T-DNA from *Agrobacterium tumefaciens* (Zambryski, 1988) |

**REFERENCES**

| |
|---|
| Christensen A. H., Sharrock R.A., Quail P.H. (1992). Maize polyubiquitin genes: structure, thermal pertubation of expression and transcript splicing, and promoter activity following transfer to protoplasts by electroporation. Plant Molecular Biology, 18, 675-689. |
| Depicker A., Stachel S., Dhaese P., Zambryski P., Goodman H.M. (1982). Nopaline synthase: transcript mapping and DNA sequence. Journal of Molecular and Applied Genetics, 1, 561-573. |
| Gielen J.; De Beuckeleer M.; Seurinck J.; Deboeck F.; De Greve H.; Lemmers M.; Van Montagu M.; Schell J. (1984). Isolation of an efficient actin promoter for use in rice transformation. The EMBO journal, 3, 835-846 |
| Hwang Y.-S., Yang D., McCullar C., Wu L., Chen L., Pham P., Nandi S., Huang N. (2002). Analysis of the rice-endosperm-specific globulin promoter in transformed rice cells. Plant Cell Rep **20**, 842-847. |
| Leroux B., Pelissier B., Lebrun M. (1996). Chimeric herbicide resistance gene. US Patent US5559024 (24-SEPT-1996), RHONE POULENC AGROCHIMIE (FR). |
| Mc Elroy D., Zhang W., Cao J., Wu R. (1990). Isolation of an efficient actin promoter for use in rice transformation. The Plant Cell, 2, 163-171. |
| Thompson C.J., Rao Mowa N., Tizard R., Crameri R., Davies J., Lauwereys M., Botterman J. (1987). Characterization of the herbicide resistance gene bar from Streptomyces hygroscopicus. The EMBO Journal, 6, 2519-2523. |
| Zambryski P. (1988). Basic processes underlying Agrobacterium-mediated DNA transfer to plant cells. Annual Review of Genetics, 22, 1-30. |

**Example 2: Production and identification of genetically modified corn plants which have an increased SSII activity**

For production of genetically modified plants having an increased starch synthase II (SSII) activity, the T-DNA of plasmid pJH77 as described in Ishida et al. (1996, Nature Biotechnology 14 (6): 745-750) was transferred to corn plants using agrobacteria. The increase in SS2 activity was demonstrated in zymograms.

Figure 2 shows zymograms of two genetically modified corn lines on the basis of which the SS2 activity was determined in comparison with the wild type. For this, total protein was extracted from unripe kernels (harvested 15 days after pollination), both from the wild type and also from the transgenic lines. The protein extracts of the transgenic lines were applied in a dilution series and thus the level of activity was compared with the intensity of the SS2 band of the wild type. The SS2 activity has here increased 5x.

**Example 3: Analysis of the starches and flours of genetically modified corn plants which have an increased SSII activity**

Flours were produced from individual kernels. The individual kernels were analyzed by PCR. Kernels in which the SSII from wheat is present (hereinafter termed "transgenic") and kernels which do not express SSII from wheat (hereinafter termed "wild type") were identified, separated from one another and then in each case combined to form groups and flours were produced. The analysis was subsequently performed on these flours. For the production of starches, in each case 10 kernels of the wild type plants and 10 kernels of the transgenic corn plants were combined and starch was isolated in each case therefrom.

The starches/flours of the transgenic heterozygotic corn lines, compared with the starches/flours of the wild type corn plants have an unchanged amylose content, an increased DSC T-onset temperature, an increased DSC T-peak temperature, an increased RS content and also a decreased RDS content.

In addition, the starches/flours of the transgenic heterozygotic corn lines, compared with the starches/flours of the wild type corn plants, are distinguished by an altered side chain distribution of the amylopectin:

a. Amylose content of the starch:

| Sample | Amylose content in % by weight |
|---|---|
| Wild type plant (A188) | 24.8 |
| JH77-00701-2 | 26.0 |
| JH77-02101 | 24.1 |

b. DSC-data of the extracted starch (DSC analysis with 2-fold water excess)

| Sample | T onset in ˚C | T peak in ˚C |
|---|---|---|
| Wild type plant (A188) | 66.82 | 73.41 |
| JH77-00701-2 | 71.13 | 78.39 |
| JH77-02101 | 70.97 | 78.22 |
| Hylon® 7 | 68.42 | 77.87 |

c. RS, SDS and RDS content of the extracted starch:

| Sample | RS (in % by weight) | SDS (in % by weight) | RDS (in % by weight) |
|---|---|---|---|
| Wild type plant (A188) | 10.5 | 62.3 | 27.2 |
| JH77-00701-2 | 46.2 | 43.8 | 10.0 |
| JH77-02101 | 42.5 | 46.2 | 11.3 |
| Hylon® 7 | 74.9 | 16.0 | 9.0 |

d. Side chain distribution of the amylopectin

| Sample | DP6-11 (sum of the areas in the chromatogram) | Ratio to the wild type in % | Difference from the wild type (in %) |
|---|---|---|---|
| Wild type plant (A188) | 20.2 | 100% | 0% |
| JH77-00701-2 | 17.4 | 86.2 | -13.8 |
| JH77-02101 | 18.1 | 89.6 | -10.4 |
| Wild type plant (A188) | 21.6 | 100% | 0% |
| JH77-00701-2 | 23.2 | 107.6 | 7.6 |
| JH77-02101 | 23.5 | 108.9 | 8.9 |

## Claims

**1.** A corn starch which has

- an apparent amylose content between 15% by weight and 40% by weight, determined according to the method of Juliano, 1971, Cereal Science Today 16, 10, p. 334-340, and
- a content of resistant starch (RS), determined by the method of Englyst et al., Europ. J. of Clinical Nutrition 46, Suppl. 2, 1992, p. 33-50, of between 15% by weight and 60% by weight, and
- a content of rapidly digestible starch (RDS) based on the amount of starch in dry weight of between 5% by weight and 25% by weight, wherein the content of RDS is the fraction of a corn starch which is released as glucose after 20 minutes in the method of Englyst et al. for determining the RS content and,

- a content of slowly digestible starch (SDS) based on the amount of starch in dry weight of between 20% by weight and 60% by weight, wherein the content of SDS is the fraction of a corn starch which is released as glucose after 2 hours in the method of Englyst et al. minus the glucose released after 20 minutes,

wherein the starch is obtained by extraction from a corn plant which expresses a heterologous starch synthase II from the genus triticum.

**2.** The corn starch as claimed in claim 1 which has an apparent amylose content between 18% by weight and 35% by weight.

**3.** The corn starch as claimed in claim 1 which has an apparent amylose content between 20% by weight and 30% by weight.

**4.** The corn starch as claimed in one of claims 1-3 which has a rapidly digestible starch (RDS) content between 10% by weight and 23% by weight.

**5.** The corn starch as claimed in one of claims 1-3 which has a rapidly digestible starch (RDS) content between 10% by weight and 17% by weight.

**6.** The corn starch as claimed in one of claims 1 to 5 which is granular.

**7.** The corn starch as claimed in claim 1 or 6, wherein the amino acid sequence of the heterologous starch synthase II has an identity of at least 86% with amino acids 322 to 351 of the amino acid sequence represented under SEQ ID No. 2 and an identity of at least 83% with amino acids 423 to 462 of the amino acid sequence represented under SEQ ID No. 2 and an identity of at least 70% with amino acids 641 to 705 of the amino acid sequence represented under SEQ ID No. 2.

**8.** The use of corn starch as claimed in one of claims 1 to 7 as prebiotic.

**9.** The use of corn starch as claimed in one of claims 1 to 7 for producing a food, which has a reduced glycemic index compared with the glycemic index of a food which comprises starch from corresponding wild type corn plants.

**10.** The use of corn starch as claimed in one of claims 1 to 7 for decreasing the glycemic index of foods comprising said corn starch compared with the glycemic index of foods which comprise starch from corresponding wild type corn plants.

**11.** A corn flour comprising corn starch as claimed in one of claims 1 to 7.

**12.** The use of corn flour as claimed in claim 11 as prebiotic.

**13.** The use of corn flour as claimed in claim 11 for producing a food, which has a reduced glycemic index compared with the glycemic index of a food which comprises flour from corresponding wild type corn plants.

**14.** The use of corn flour as claimed in claim 11 for decreasing the glycemic index of foods comprising said corn flour compared with the glycemic index of foods which comprise the flour from corresponding wild type corn plants.

**15.** A composition comprising corn starch as claimed in one of claims 1 to 7 and at least one food additive.

**16.** A composition comprising corn flour as claimed in claim 11 and at least one food additive.

**17.** A food comprising corn starch as claimed in one of claims 1 to 7, which has a reduced glycemic index compared with the glycemic index of a food which comprises starch from corresponding wild type corn plants.

**18.** A food comprising corn flour as claimed in claim 11, which has a reduced glycemic index compared with the glycemic index of a food which comprises flour from corresponding wild type corn plants.

**19.** A food comprising a composition as claimed in either of claims 15 or 16.

## Patentansprüche

1. Maisstärke mit

   - einem scheinbaren Amylosegehalt zwischen 15 Gew.-% und 40 Gew.-%, bestimmt nach dem Verfahren von Juliano, 1971, Cereal Science Today 16, 10, S. 334-340, und
   - einem Gehalt an resistenter Stärke (RS), bestimmt nach dem Verfahren von Englyst et al., Europ. J. of Clinical Nutrition 46, Ergänzungsband 2, 1992, S. 33-50, von zwischen 15 Gew.-% und 60 Gew.-% und
   - einem Gehalt an rasch verdaulicher Stärke (RDS) von zwischen 5 Gew.-% und 25 Gew.-% in Bezug auf die Stärkemenge im Trockengewicht, wobei der RDS-Gehalt denjenigen Anteil einer Maisstärke, der in der Methode von Englyst et al. für die Bestimmung des RS-Gehalts nach 20 Minuten als Glucose freigesetzt wird, darstellt, und
   - einem Gehalt an langsam verdaulicher Stärke (SDS) von zwischen 20 Gew.-% und 60 Gew.-% in Bezug auf die Stärkemenge im Trockengewicht, wobei der SDS-Gehalt denjenigen Anteil einer Maisstärke, der in dem Verfahren von Englyst et al. nach 2 Stunden als Glucose freigesetzt wird, minus der Glucose, die nach 20 Minuten freigesetzt wird, darstellt,

   wobei die Stärke durch Extrahieren aus einer Maispflanze, die eine heterologe Stärkesynthase II aus der Gattung Triticum exprimiert, erhalten wird.

2. Maisstärke nach Anspruch 1, die einen scheinbaren Amylosegehalt zwischen 18 Gew.-% und 35 Gew.-% aufweist.

3. Maisstärke nach Anspruch 1, die einen scheinbaren Amylosegehalt zwischen 20 Gew -% und 30 Gew.-% aufweist.

4. Maisstärke nach einem der Ansprüche 1-3, die einen Gehalt an rasch verdaubarer Stärke (RDS) zwischen 10 Gew.-% und 23 Gew.-% aufweist.

5. Maisstärke nach einem der Ansprüche 1-3, die einen Gehalt an rasch verdaubarer Stärke (RDS) zwischen 10 Gew.-% und 17 Gew.-% aufweist.

6. Maisstärke nach einem der Ansprüche 1 bis 5, die granulatförmig ist.

7. Maisstärke nach Anspruch 1 oder 6, wobei die Aminosäuresequenz der heterologen Stärkesynthase II eine Identität von mindestens 86% zu den Aminosäuren 322 bis 351 der Aminosäuresequenz gemäß SEQ ID No. 2 und eine Identität von mindestens 83% mit den Aminosäuren 423 bis 462 der Aminosäuresequenz gemäß SEQ ID No. 2 und eine Identität von mindestens 70% zu den Aminosäuren 641 bis 705 der Aminosäuresequenz gemäß SEQ ID No. 2 aufweist.

8. Verwendung einer Maisstärke nach einem der Ansprüche 1 bis 7 als Präbiotikum.

9. Verwendung einer Maisstärke nach einem der Ansprüche 1 bis 7 für die Herstellung eines Lebensmittels, das einen verringerten glykämischen Index im Vergleich zu dem glykämischen Index eines Lebensmittels, das Stärke aus entsprechenden Wildtypmalspflanzen umfasst, aufweist.

10. Verwendung einer Maisstärke nach einem der Ansprüche 1 bis 7 für die Verringerung des glykämischen Index von Lebensmitteln umfassend diese Maisstärke im Vergleich zu dem glykämischen Index von Lebensmitteln, die Stärke aus den entsprechenden Wildtypmaispflanzen umfassen.

11. Maismehl, umfassend Maisstärke nach einem der Ansprüche 1 bis 7.

12. Verwendung von Maismehl nach Anspruch 11 als Präbiotikum,

13. Verwendung von Maismehl nach Anspruch 11 für die Herstellung eines Lebensmittels, das einen verringerten glykämischen Index im Vergleich zu dem glykämischen Index eines Lebensmittels, das Mehl aus entsprechenden Wildtypmaispflanzen umfasst, aufweist.

14. Verwendung von Maismehl nach Anspruch 11 für die Verringerung des glykämischen Index von Lebensmitteln umfassend dieses Maismehl im Vergleich zu dem glykämischen Index von Lebensmitteln, die Mehl aus den entsprechenden Wildtypmaispflanzen umfassen.

**15.** Zusammensetzung, umfassend Maisstärke nach einem der Ansprüche 1 bis 7 und mindestens einen Lebensmittelzusatzstoff.

**16.** Zusammensetzung, umfassend Maismehl nach Anspruch 11 und mindestens einen Lebensmittelzusatzstoff.

**17.** Lebensmittel, umfassend Maisstärke nach einem der Ansprüche 1 bis 7, das einen verringerten glykämischen Index im Vergleich zu dem glykämischen Index eines Lebensmittels, das Stärke aus entsprechenden Wildtypmaispflanzen umfasst, aufweist.

**18.** Lebensmittel, umfassend Maismehl nach Anspruch 11, das einen verringerten glykämischen Index im Vergleich zu dem glykämischen Index eines Lebensmittels, das Mehl aus entsprechenden Wildtypmaispflanzer umfasst, aufweist.

**19.** Lebensmittel, umfassend eine Zusammensetzung nach einem der Ansprüche 15 oder 16.

## Revendications

**1.** Amidon de maïs, présentant:

- une teneur apparente en amylose qui est comprise entre 15 % en poids et 40 % en poids, déterminée selon la méthode de Juliano, 1971, Cereal Science Today 16, 10, pages 334 à 340; et
- une teneur en amidon résistant (AR), déterminée par la méthode d'Englyst et al., Europ. J. of Clinical Nutrition 46, Suppl. 2, 1992, pages 33 à 50, qui est comprise entre 15 % en poids et 60 % en poids; et
- une teneur en amidon rapidement digestible (ARD), basée sur la quantité d'amidon en poids à sec, qui est comprise entre 5 % en poids et 25 % en poids, dans lequel la teneur en ARD correspond à la fraction d'un amidon de maïs qui est libéré sous forme de glucose après 20 minutes selon la méthode d'Englyst et al. pour déterminer la teneur en AR; et
- une teneur en amidon lentement digestible (ALD), basée sur la quantité d'amidon en poids à sec, qui est comprise entre 20 % en poids et 60 % en poids, dans lequel la teneur en ALD correspond à la fraction d'un amidon de maïs qui est libéré sous forme de glucose après 2 heures dans la méthode d'Englyst et al. moins le glucose libéré après 20 minutes,

dans lequel l'amidon est obtenu par extraction à partir d'un plant de maïs qui exprime une synthase d'amidon hétérologue II à partir du *genus triticum.*

**2.** Amidon de maïs selon la revendication 1, qui présente une teneur apparente en amylose qui est comprise entre 18 % en poids et 35 % en poids.

**3.** Amidon de maïs selon la revendication 1, qui présente une teneur apparente en amylose qui est comprise entre 20 % en poids et 30 % en poids.

**4.** Amidon de maïs selon l'une quelconque des revendications 1 à 3, qui présente une teneur en amidon rapidement digestible (ARD) qui est comprise entre 10 % en poids et 23 % en poids.

**5.** Amidon de mais selon l'une quelconque des revendications 1 à 3, qui présente une teneur en amidon rapidement digestible (ARD) qui est comprise entre 10 % en poids et 17 % en poids.

**6.** Amidon de maïs selon l'une quelconque des revendications 1 à 5, qui est granulaire.

**7.** Amidon de maïs selon la revendication 1 ou 6, dans lequel la séquence d'acides aminés de la synthase d'amidon hétérologue II présente une identité d'au moins 86 % avec les acides aminés 322 à 351 de la séquence d'acides aminés représentée par SEQ ID n° 2, et une identité d'au moins 83 % avec les acides aminés 423 à 462 de la séquence d'acides aminés représentée par SEQ ID n° 2, et une identité d'au moins 70 % avec les acides aminés 641 à 705 de la séquence d'acides aminés représentée par SEQ ID n° 2.

**8.** Utilisation de l'amidon de maïs selon l'une quelconque des revendications 1 à 7 comme prébiotique.

**9.** Utilisation d'amidon de maïs selon l'une quelconque des revendications 1 à 7 pour produire un produit alimentaire, qui présente un indice glycémique réduit comparativement à l'indice glycémique d'un produit alimentaire qui contient de l'amidon en provenance de plants de maïs correspondants de type sauvage.

**10.** Utilisation d'amidon de maïs selon l'une quelconque des revendications 1 à 7 pour diminuer l'indice glycémique de produits alimentaires qui contiennent ledit amidon de maïs comparativement à l'indice glycémique d'un produit alimentaire qui contient de l'amidon en provenance de plants de maïs correspondants de type sauvage.

**11.** Farine de maïs contenant de l'amidon de maïs selon l'une quelconque des revendications 1 à 7.

**12.** Utilisation de la farine de maïs selon la revendication 11 comme prébiotique.

**13.** Utilisation de la farine de maïs selon la revendication 11 pour produire un produit alimentaire, qui présente un indice glycémique réduit comparativement à l'indice glycémique d'un produit alimentaire qui contient de la farine en provenance de plants de maïs correspondants de type sauvage.

**14.** Utilisation de la farine de maïs selon la revendication 11 pour diminuer l'indice glycémique de produits alimentaires contenant ladite farine de maïs comparativement à l'indice glycémique de produits alimentaires qui contiennent de la farine en provenance de plants de maïs correspondants de type sauvage.

**15.** Composition contenant de la farine de maïs selon l'une quelconque des revendications 1 à 7 et au moins un additif alimentaire.

**16.** Composition contenant de la farine de maïs selon la revendication 11 et au moins un additif alimentaire.

**17.** Produit alimentaire contenant de l'amidon de maïs selon l'une quelconque des revendications 1 à 7, qui présente un indice glycémique réduit comparativement à l'indice glycémique d'un produit alimentaire qui contient de l'amidon en provenance de plants de maïs correspondants de type sauvage.

**18.** produit alimentaire contenant de l'amidon de maïs selon la revendication 11, qui présente un indice glycémique réduit comparativement à l'indice glycémique d'un produit alimentaire qui contient de l'amidon en provenance de plants de maïs correspondants de type sauvage.

**19.** Produit alimentaire contenant une composition selon l'une ou l'autre des revendications 15 ou 16.

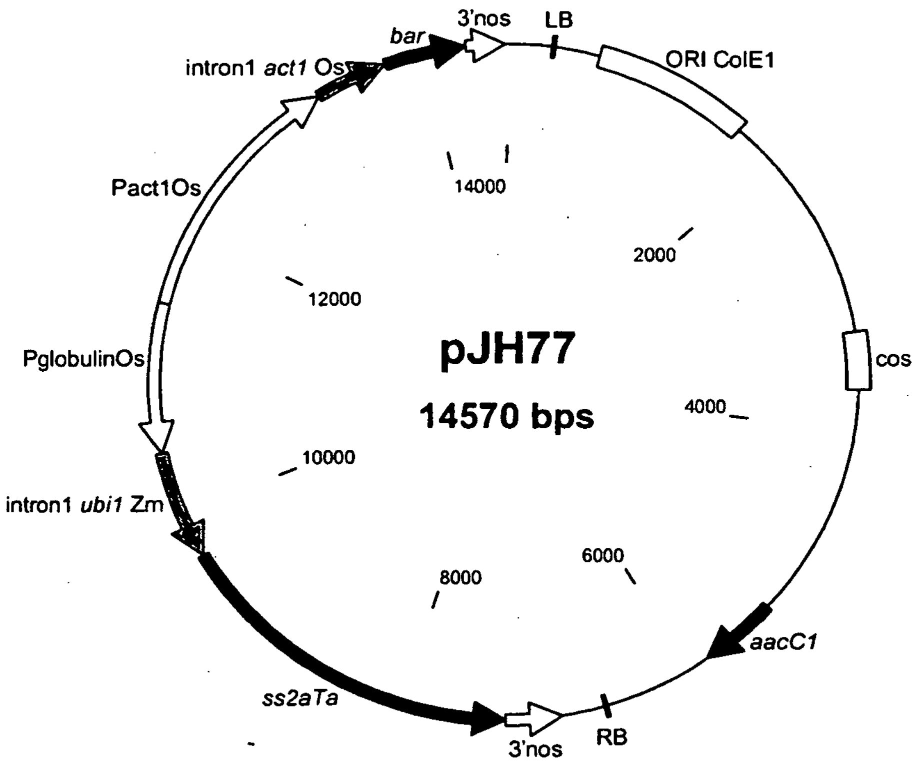
3'nos
LB
bar
intron1 act1 Os
ORI ColE1
14000
Pact1Os
2000
12000
PglobulinOs
pJH77
14570 bps
cos
4000
10000
intron1 ubi1 Zm
6000
8000
aacC1
ss2aTa
3'nos
RB

Figure 1

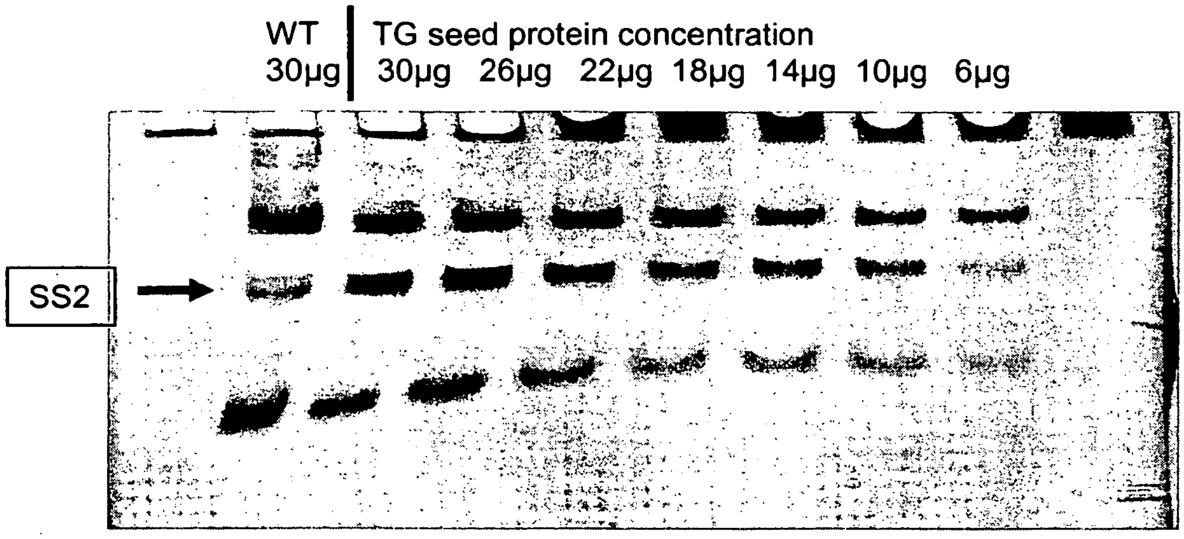


Figure 2: Zymogram

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 564893 A1
- EP 688872 A1
- EP 846704 A1
- US 5051271 A
- US 6750378 B
- WO 0173087 A
- WO 9745545 A
- US 5559024 A
- EP 06090228 A
- EP 07075964 A
- US 61002390 B
- US 60879377 B


### Non-patent literature cited in the description


- **Hizukuri ; Takagi.** *Carbohydr. Res.,* 1984, vol. 134, 1-10
- **Takeda et al.** *Carbohydr. Res.,* 1984, vol. 132, 83-92
- **Banks ; Greenwood.** Starch and its components. Edinburgh University Press, 51-66
- **Larson et al.** *Analytical Chemistry,* 1953, vol. 25 (5), 802-804
- **Morrison ; Laignelet.** *J. Cereal Sc.,* 1983, vol. 1, 9-20
- **Kugimiya ; Donovan.** *Journal of Food Science,* 1981, vol. 46, 765-770
- **Sievert ; Holm.** *Starch/Stärke,* 1993, vol. 45 (4), 136-139
- **Faisant et al.** *Sciences des Aliments,* 1995, vol. 15, 83-89
- **Evans ; Thompson.** *Cereal Chemistry,* 2004, vol. 81 (1), 31-37
- **Englyst et al.** *Europ. J. of Clinical Nutrition,* 1992, vol. 46 (2), 33-50
- **McCleary ; Monaghan.** *J. AOAC Int.,* 2002, vol. 85, 665-675
- **Senti ; Russell.** *STARCH/STÄRKE,* April 1960, vol. 43 (4), 343-349
- **Z. Luo et al.** *Starch/Stärke,* 2006, vol. 58, 468-474
- **Englyst et al.** *Eur. J. Clin. Nutr.,* 1992, vol. 46, S33-S50
- **Faisant et al.** *Sci. Aliment.,* 1995, vol. 15, 83-89
- **Champ et al.** Advanced Dietary Fibre Technology. Blackwell Science Ltd, 106-119
- **Goni et al.** *Food Chem.,* 1996, vol. 56, 445-449
- **Berry.** *J. Cereal Sci.,* 1986, vol. 4, 301-314
- **McCleary et al.** *J. AOAC Int.,* 2002, vol. 85, 1103-1111
- Approved Methods of the American Association of Cereal Chemists. AACC, 2000, vol. I
- **Baghurst et al.** *Supplement to Food Australia,* 1996, vol. 48 (3), S3-S35
- **Delcour ; Eerlingen.** *Cereal Foods World,* 1996, vol. 41 (2), 85-86
- **Englyst et al.** *British Journal of Nutrition,* vol. 75, 327-337
- **Juliano.** *Cereal Science Today,* 1971, vol. 16 (10), 334-340
- **Englyst et al.** *Europ. J. Clinical Nutrition,* 1992, vol. 46 (2), 33-50
- **Yahl.** *Microscope,* 1984, vol. 32, 123-132
- **Munster et al.** *Digestive Diseases and Sciences,* 1994, vol. 39 (4), 834-842
- **Wolever et al.** *Am. J. Clin. Nutr.,* 1991, vol. 54, 846-854
- Carbohydrates in human nutrition. The Role of the Glycemic Index in Food Choice. 14 April 1997, 25-30
- **Starch.** Chemistry and Technology. Academic Press Inc, 1994, 412-468
- **Watson; Eckhoff et al.** *Cereal Chem.,* 1998, vol. 73, 54-57
- **Li et al.** *Funct Integr Genomics,* 2003, vol. 3, 76-85
- **Thompson et al.** *Nucleic Acids Research,* 1994, vol. 22, 4673-4680
- **Gao ; Chibbar.** *Genome,* 2000, vol. 43 (5), 768-775
- **Nishi et al.** *Plant Physiology,* 2001, vol. 127, 459-472
- **Odell et al.** *Nature,* 1985, vol. 313, 810-812
- **Christensen.** *Plant Mol. Biol.,* 1992, vol. 18, 675-689
- **Liu et al.** *Plant Science,* 2003, vol. 165
- **Zhang et al.** *Plant Cell,* 1991, vol. 3, 1150-1160
- **Verdaguer.** *Plant Mol. Biol.,* vol. 31, 1129-1139

- **Stavolone et al.** *Plant Mol. Biol.,* 2003, vol. 53, 703-713
- **Kirihara et al.** *Gene,* 1988, vol. 71, 359-370
- **Hoffmann et al.** *EMBO J.,* 1987, vol. 6, 3213-3221
- **Schernthaner et al.** *EMBO J.,* 1988, vol. 7, 1249-1253
- **Williamson et al.** *Plant Physiol.,* 1988, vol. 88, 1002-1007
- **Prat et al.** *Gene,* 1987, vol. 52, 51-49
- **Gallardo et al.** *Plant Sci.,* 1988, vol. 54, 211-281
- **Marks et al.** *J. Biol. Chem.,* 1985, vol. 260, 16451-16459
- **Kodrzyck et al.** *Plant Cell,* 1989, vol. 1, 105-114
- **Yang, N.-S. ; Russel, D.** *Proc. Natl. Acad Sci,* 1990, vol. 87, 4144-4148
- **Unger et al.** *Plant Physiol.,* 1991, vol. 96, 124
- **Bhave et al.** *Plant Cell,* 1990, vol. 2, 581-588
- **Bae et al.** *Maydica,* 1990, vol. 35, 317-322
- **Colot et al.** *EMBO J.,* 1987, vol. 6, 3559-3564
- **Clarke ; Appels.** *Genome,* 1998, vol. 41, 865-871
- **Pedersen et al.** *Cell,* 1982, vol. 29, 1015-1026
- **Quatroccio et al.** *Plant Mol. Biol.,* 1990, vol. 15, 81-93
- **Leisy et al.** *Plant Mol. Biol.,* 1990, vol. 14, 41-50
- **Zheng et al.** *Plant J,* 1993, vol. 4, 357-366
- **Yoshihara et al.** *FEBS Lett.,* 1996, vol. 383, 213-218
- **Nakase et al.** *Gene,* 1996, vol. 170 (2), 223-226
- **Qu ; Takaiwa.** *Plant Biotechnology Journal,* 2004, vol. 2 (2), 113-125
- **Callis et al.** *Genes Devel.,* 1987, vol. 1, 1183-1200
- **Luehrsen ; Walbot.** *Mol. Gen. Genet.,* 1991, vol. 225, 81-93
- **Rethmeier et al.** *Plant Journal.,* 1997, vol. 12 (4), 895-899
- **Rose ; Beliakoff.** *Plant Physiol.,* 2000, vol. 122 (2), 535-542
- **Vasil et al.** *Plant Physiol.,* 1989, vol. 91, 1575-1579
- **XU et al.** *Science in China Series C,* 2003, vol. 46 (6), 561-569
- **Maas et al.** *Plant. Mol. Biol.,* 1991, vol. 16, 199-207
- **Callis et al.** *Genes Dev.,* 1987, vol. 1, 1183-1200
- **Luehrsen, K. R. ; Walbot, V.** *Mol. Gen. Genet.,* 1991, vol. 225, 81-93
- **Oard et al.** *Plant Cell Rep,* 1989, vol. 8, 156-160
- **Sambrok et al.** Molecular Cloning, A Laboratory Manual. Cold Spring Harbour Laboratory Press, 2001
- **Ausubel et al.** Short Protocols in Molecular Biology. John Wiley & Sons, 2002
- **Sanders et al.** *Cereal Chemistry,* 1990, vol. 67 (6), 594-602
- **Fisher et al.** *Plant Physiol.,* 1996, vol. 110, 611-619
- **Hedman ; Boyer.** *Biochemical Genetics,* 1983, vol. 21 (11/12), 1217-1222
- **Kim et al.** *Plant Molecular Biology,* 1998, vol. 38, 945-956
- **Guan ; Preiss.** *Plant Physiol.,* 1993, vol. 102, 1269-1273
- **Gao et al.** *Plant Physiol.,* 1997, vol. 114, 69-78
- **Stinard et al.** *Plant Cell,* 1993, vol. 5, 1555-1566
- **Hedman ; Boyer.** *Biochemical genetics,* 1982, vol. 20 (5/6), 483-492
- **Vineyard ; Bear.** *Maize Genet Coop Newsletter,* 1952, vol. 26, 5
- **Moore ; Creech.** *Genetics,* 1972, vol. 70, 611-619
- **Garwood et al.** *Cereal Chemistry,* 1976, vol. 53 (3), 355-364
- **Ishida et al.** *Nature Biotechnology,* 1996, vol. 14, 745-750
- **Bradford.** *Anal Biochem,* 1976, vol. 72, 248-254
- Tutorial and User Manual. March 2004
- **Perkin Elmer.** *Large Volume Stainless Steel Pans,* vol. 60 μl
- **Nielsen et al.** *Plant Physiol.,* 1994, vol. 105, 111-117
- **Englyst et al.** *Reagents, Apparatus, Spectrophotometer,* S35-S36
- *Measurement of free glucose (FG),* S36-S37
- *Measurement of RDS and SDS,* S38
- **Zhang et al.** *Biomacromolecules,* 2006, vol. 7, 3252-3258
- **Christensen A. H. ; Sharrock R.A. ; Quail P.H.** Maize polyubiquitin genes: structure, thermal pertubation of expression and transcript splicing, and promoter activity following transfer to protoplasts by electroporation. *Plant Molecular Biology,* 1992, vol. 18, 675-689
- **Depicker A. ; Stachel S. ; Dhaese P. ; Zambryski P. ; Goodman H.M.** Nopaline synthase: transcript mapping and DNA sequence. *Journal of Molecular and Applied Genetics,* 1982, vol. 1, 561-573
- **Gielen J. ; De Beuckeleer M. ; Seurinck J. ; Deboeck F. ; De Greve H. ; Lemmers M. ; Van Montagu M. ; Schell J.** Isolation of an efficient actin promoter for use in rice transformation. *The EMBO journal,* 1984, vol. 3, 835-846

- **Hwang Y.-S. ; Yang D. ; McCullar C. ; Wu L. ; Chen L. ; Pham P. ; Nandi S.** Analysis of the rice-endosperm-specific globulin promoter in transformed rice cells. *Plant Cell Rep,* 2002, vol. 20, 842-847
- **Mc Elroy D. ; Zhang W. ; Cao J. ; Wu R.** Isolation of an efficient actin promoter for use in rice transformation. *The Plant Cell,* 1990, vol. 2, 163-171
- **Thompson C.J. ; Rao Mowa N. ; Tizard R. ; Crameri R. ; Davies J. ; Lauwereys M. ; Botterman J.** Characterization of the herbicide resistance gene bar from Streptomyces hygroscopicus. *The EMBO Journal,* 1987, vol. 6, 2519-2523
- **Zambryski P.** Basic processes underlying Agrobacterium-mediated DNA transfer to plant cells. *Annual Review of Genetics,* 1988, vol. 22, 1-30
- **Ishida et al.** *Nature Biotechnology,* 1996, vol. 14 (6), 745-750

SEQUENCE LISTING

<110> Bayer CropScience AG
<120> Corn starch and also corn flours and food comprising this corn starch
<130> BCS 06-5014 PCT
<150> EP 06090228.5
<151> 2006-12-29
<150> EP 07075964.2
<151> 2007-11-06
<150> US 61/002,390
<151> 2007-11-08
<150> US 60/879,377
<151> 2007-01-09
<160> 8
<170> PatentIn version 3.3
<210> 1
<211> 2400
<212> DNA
<213> Triticum aestivum
<400> 1

```
atgtcgtcgg cggtcgcgtc cgccgcatcc ttcctcgcgc tcgcgtcagc ctcccccggg    60
agatcacgca ggcgggcgag ggtgagcgcg cagccacccc acgccggggc cggcaggttg   120
cactggccgc cgtggccgcc gcagcgcacg gctcgcgacg gagctgtggc ggcgctcgcc   180
gccgggaaga aggacgcggg gatcgacgac gccgccgcgt ccgtgaggca gccccgcgca   240
ctccgcggtg gcgccgccac caaggtcgcg gagcgaaggg atcccgtcaa gacgctcgac   300
cgcgacgccg cggaaggcgg cgggccgtcc ccgccggcag cgaggcagga cgccgcccgt   360
ccgccgagta tgaacggcat gccggtgaac ggcgagaaca aatctaccgg cggcggcggc   420
gcgactaaag acagcgggct gcccacgccc gcacgcgcgc cccatccgtc gacccagaac   480
agagcaccgg tgaacggtga aaacaaagct aacgtcgcct cgccgccgac gagcatagcc   540
gaggccgcgg cttcggattc cgcagctacc atttccatca gcgacaaggc gccggagtcc   600
gttgtcccag ctgagaagac gccgccgtcg tccggctcaa atttcgagtc ctcggcctct   660
gctcccgggt ctgacactgt cagcgacgtg gaacaagaac tgaagaaggg tgcggtcgtt   720
```

gtcgaagaag ctccaaagcc aaaggctctt tcgccgcctg cagccccccgc tgtacaagaa 780

gacctttggg atttcaagaa atacattggt ttcgaggagc ccgtggaggc caaggatgat 840

ggccgggctg tcgcagatga tgcgggctcc tttgaacacc accagaatca cgactccgga 900

cctttggcag gggagaatgt catgaacgtg gtcgtcgtgg ctgctgagtg ttctccctgg 960

tgcaaaacag gtggtctggg agatgttgcg ggtgctctgc ccaaggcttt ggcaaagaga 1020

ggacatcgtg ttatggttgt ggtaccaagg tatggggact atgaagaagc ctacgatgtc 1080

ggagtccgaa aatactacaa ggctgctgga caggatatgg aagtgaatta tttccatgct 1140

tatatcgatg gagttgattt tgtgttcatt gacgctcctc tcttccgaca ccgtcaggaa 1200

gacatttatg gggcagcag acaggaaatt atgaagcgca tgattttgtt ctgcaaggcc 1260

gctgttgagg ttccatggca cgttccatgc ggcggtgtcc cttatgggga tggaaatctg 1320

gtgtttattg caaatgattg gcacacggca ctcctgcctg tctatctgaa agcatattac 1380

agggaccatg gtttgatgca gtacactcgg tccattatgg tgatacataa catcgctcac 1440

cagggccgtg gccctgtaga tgaattcccg ttcaccgagt tgcctgagca ctacctggaa 1500

cacttcagac tgtacgaccc cgtgggtggt gaacacgcca actacttcgc cgccggcctg 1560

aagatggcgg accaggttgt cgtggtgagc ccccgggtacc tgtgggagct gaagacggtg 1620

gagggcggct gggggcttca cgacatcata cggcagaacg actggaagac ccgcggcatc 1680

gtcaacggca tcgacaacat ggagtggaac cccgaggtgg acgcccacct caagtcggac 1740

ggctacacca acttctccct gaggacgctg gactccggca agcggcagtg caaggaggcc 1800

ctgcagcgcg agctgggcct gcaggtccgc gccgacgtgc cgctgctcgg cttcatcggc 1860

cgcctggacg ggcagaaggg cgtggagatc atcgcggacg ccatgccctg gatcgtgagc 1920

caggacgtgc agctggtgat gctgggcacc gggcgccacg acctggagag catgctgcag 1980

cacttcgagc gggagcacca cgacaaggtg cgcgggtggg tggggttctc cgtgcgcctg 2040

gcgcaccgga tcacggcggg ggcggacgcg ctcctcatgc cctcccggtt cgagccgtgc 2100

gggctgaacc agctctacgc catggcctac ggcaccgtcc ccgtcgtgca cgccgtcggc 2160

ggcctcaggg acaccgtgcc gccgttcgac cccttcaacc actccgggct cgggtggacg 2220

ttcgaccgcg ccgaggcgca caagctgatc gaggcgctcg ggcactgcct ccgcacctac 2280

cgagacttca aggagagctg gagggccctc caggagcgcg gcatgtcgca ggacttcagc 2340

tgggagcacg ccgccaagct ctacgaggac gtcctcgtca aggccaagta ccagtggtga 2400

<210> 2
<211> 799
<212> PRT
<213> Triticum aestivum
<400> 2

```
Met Ser Ser Ala Val Ala Ser Ala Ala Ser Phe Leu Ala Leu Ala Ser
1               5                   10                  15

Ala Ser Pro Gly Arg Ser Arg Arg Arg Ala Arg Val Ser Ala Gln Pro
            20                  25                  30

Pro His Ala Gly Ala Gly Arg Leu His Trp Pro Pro Trp Pro Pro Gln
        35                  40                  45

Arg Thr Ala Arg Asp Gly Ala Val Ala Ala Leu Ala Ala Gly Lys Lys
    50                  55                  60

Asp Ala Gly Ile Asp Asp Ala Ala Ala Ser Val Arg Gln Pro Arg Ala
65                  70                  75                  80

Leu Arg Gly Gly Ala Ala Thr Lys Val Ala Glu Arg Arg Asp Pro Val
                85                  90                  95

Lys Thr Leu Asp Arg Asp Ala Ala Glu Gly Gly Gly Pro Ser Pro Pro
            100                 105                 110

Ala Ala Arg Gln Asp Ala Ala Arg Pro Pro Ser Met Asn Gly Met Pro
        115                 120                 125

Val Asn Gly Glu Asn Lys Ser Thr Gly Gly Gly Gly Ala Thr Lys Asp
    130                 135                 140

Ser Gly Leu Pro Thr Pro Ala Arg Ala Pro His Pro Ser Thr Gln Asn
145                 150                 155                 160

Arg Ala Pro Val Asn Gly Glu Asn Lys Ala Asn Val Ala Ser Pro Pro
                165                 170                 175
```

Thr Ser Ile Ala Glu Ala Ala Ala Ser Asp Ser Ala Ala Thr Ile Ser
180 185 190

Ile Ser Asp Lys Ala Pro Glu Ser Val Val Pro Ala Glu Lys Thr Pro
195 200 205

Pro Ser Ser Gly Ser Asn Phe Glu Ser Ser Ala Ser Ala Pro Gly Ser
210 215 220

Asp Thr Val Ser Asp Val Glu Gln Glu Leu Lys Lys Gly Ala Val Val
225 230 235 240

Val Glu Glu Ala Pro Lys Pro Lys Ala Leu Ser Pro Pro Ala Ala Pro
245 250 255

Ala Val Gln Glu Asp Leu Trp Asp Phe Lys Lys Tyr Ile Gly Phe Glu
260 265 270

Glu Pro Val Glu Ala Lys Asp Asp Gly Arg Ala Val Ala Asp Asp Ala
275 280 285

Gly Ser Phe Glu His His Gln Asn His Asp Ser Gly Pro Leu Ala Gly
290 295 300

Glu Asn Val Met Asn Val Val Val Val Ala Ala Glu Cys Ser Pro Trp
305 310 315 320

Cys Lys Thr Gly Gly Leu Gly Asp Val Ala Gly Ala Leu Pro Lys Ala
325 330 335

Leu Ala Lys Arg Gly His Arg Val Met Val Val Val Pro Arg Tyr Gly
340 345 350

Asp Tyr Glu Glu Ala Tyr Asp Val Gly Val Arg Lys Tyr Tyr Lys Ala
355 360 365

Ala Gly Gln Asp Met Glu Val Asn Tyr Phe His Ala Tyr Ile Asp Gly
370 375 380

Val Asp Phe Val Phe Ile Asp Ala Pro Leu Phe Arg His Arg Gln Glu

385 390 395 400

Asp Ile Tyr Gly Gly Ser Arg Gln Glu Ile Met Lys Arg Met Ile Leu
405 410 415

Phe Cys Lys Ala Ala Val Glu Val Pro Trp His Val Pro Cys Gly Gly
420 425 430

Val Pro Tyr Gly Asp Gly Asn Leu Val Phe Ile Ala Asn Asp Trp His
435 440 445

Thr Ala Leu Leu Pro Val Tyr Leu Lys Ala Tyr Tyr Arg Asp His Gly
450 455 460

Leu Met Gln Tyr Thr Arg Ser Ile Met Val Ile His Asn Ile Ala His
465 470 475 480

Gln Gly Arg Gly Pro Val Asp Glu Phe Pro Phe Thr Glu Leu Pro Glu
485 490 495

His Tyr Leu Glu His Phe Arg Leu Tyr Asp Pro Val Gly Gly Glu His
500 505 510

Ala Asn Tyr Phe Ala Ala Gly Leu Lys Met Ala Asp Gln Val Val Val
515 520 525

Val Ser Pro Gly Tyr Leu Trp Glu Leu Lys Thr Val Glu Gly Gly Trp
530 535 540

Gly Leu His Asp Ile Ile Arg Gln Asn Asp Trp Lys Thr Arg Gly Ile
545 550 555 560

Val Asn Gly Ile Asp Asn Met Glu Trp Asn Pro Glu Val Asp Ala His
565 570 575

Leu Lys Ser Asp Gly Tyr Thr Asn Phe Ser Leu Arg Thr Leu Asp Ser
580 585 590

Gly Lys Arg Gln Cys Lys Glu Ala Leu Gln Arg Glu Leu Gly Leu Gln
595 600 605

```
Val Arg Ala Asp Val Pro Leu Leu Gly Phe Ile Gly Arg Leu Asp Gly
    610                 615                 620

Gln Lys Gly Val Glu Ile Ile Ala Asp Ala Met Pro Trp Ile Val Ser
625                 630                 635                 640

Gln Asp Val Gln Leu Val Met Leu Gly Thr Gly Arg His Asp Leu Glu
                645                 650                 655

Ser Met Leu Gln His Phe Glu Arg Glu His His Asp Lys Val Arg Gly
            660                 665                 670

Trp Val Gly Phe Ser Val Arg Leu Ala His Arg Ile Thr Ala Gly Ala
        675                 680                 685

Asp Ala Leu Leu Met Pro Ser Arg Phe Glu Pro Cys Gly Leu Asn Gln
    690                 695                 700

Leu Tyr Ala Met Ala Tyr Gly Thr Val Pro Val Val His Ala Val Gly
705                 710                 715                 720

Gly Leu Arg Asp Thr Val Pro Pro Phe Asp Pro Phe Asn His Ser Gly
                725                 730                 735

Leu Gly Trp Thr Phe Asp Arg Ala Glu Ala His Lys Leu Ile Glu Ala
            740                 745                 750

Leu Gly His Cys Leu Arg Thr Tyr Arg Asp Phe Lys Glu Ser Trp Arg
        755                 760                 765

Ala Leu Gln Glu Arg Gly Met Ser Gln Asp Phe Ser Trp Glu His Ala
    770                 775                 780

Ala Lys Leu Tyr Glu Asp Val Leu Val Lys Ala Lys Tyr Gln Trp
785                 790                 795
```

<210> 3
<211> 20
<212> DNA

<213> Artificial sequence
<220>
<223> Oligonucleotide
<400> 3
gaagaagctc caaagccaaa 20
<210> 4
<211> 20
<212> DNA
<213> Artificial sequence
<220>
<223> Oligonucleotide
<400> 4
ggctcctcga aaccaatgta 20
<210> 5
<211> 27
<212> DNA
<213> Artificial sequence
<220>
<223> Oligonucleotide
<400> 5
tcttgaaatc ccaaaggtct tcttgta 27
<210> 6
<211> 20
<212> DNA
<213> Artificial sequence
<220>
<223> Oligonucleotide
<400> 6
gtcatcagct cgcgttgact 20
<210> 7
<211> 19
<212> DNA
<213> Artificial sequence
<220>
<223> Oligonucleotide
<400> 7
tcaatcggta ggagcgacg 19
<210> 8
<211> 26
<212> DNA
<213> Artificial sequence
<220>
<223> Oligonucleotide
<400> 8
acgtccctgc cctttgtaca caccgc 26